(19) Europäisches Patentamt
European Patent Office
Office européen des brevets

(11) EP 2 115 019 B2

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**10.07.2019 Patentblatt 2019/28**

(45) Hinweis auf die Patenterteilung:
**30.03.2016 Patentblatt 2016/13**

(21) Anmeldenummer: **08708327.5**

(22) Anmeldetag: **29.01.2008**

(51) Int Cl.:
***C08F 220/06*** *(2006.01)* ***C08F 2/40*** *(2006.01)*
***C08K 5/098*** *(2006.01)* ***C08K 5/13*** *(2006.01)*
***A61L 15/60*** *(2006.01)*

(86) Internationale Anmeldenummer:
**PCT/EP2008/051010**

(87) Internationale Veröffentlichungsnummer:
**WO 2008/092843 (07.08.2008 Gazette 2008/32)**

(54) **VERFAHREN ZUR HERSTELLUNG WEIßER UND FARBSTABILER WASSERABSORBIERENDER POLYMERPARTIKEL MIT HOHEM ABSORPTIONSVERMÖGEN UND HOHER FLÜSSIGKEITSLEITFÄHIGKEIT**

METHOD FOR PRODUCING WHITE AND COLOR-STABLE WATER-ABSORBING POLYMER PARTICLES HAVING HIGH ABSORBENCY AND HIGH SALINE FLOW CONDUCTIVITY

PROCÉDÉ DE FABRICATION DE PARTICULES POLYMÈRES BLANCHES, RÉSISTANTES À LA DÉCOLORATION, ABSORBANT L'EAU, PRÉSENTANT UNE GRANDE CAPACITÉ D'ABSORPTION ET UNE GRANDE CONDUCTIVITÉ DE LIQUIDES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **29.01.2007 EP 07101342**

(43) Veröffentlichungstag der Anmeldung:
**11.11.2009 Patentblatt 2009/46**

(73) Patentinhaber: **BASF SE**
**67056 Ludwigshafen am Rhein (DE)**

(72) Erfinder:
- **RIEGEL, Ulrich**
  **66849 Landstuhl (DE)**
- **DANIEL, Thomas**
  **67165 Waldsee (DE)**
- **STUEVEN, Uwe**
  **65812 Bad Soden (DE)**
- **ELLIOTT, Mark**
  **67063 Ludwigshafen (DE)**
- **BRAIG, Volker**
  **69469 Weinheim-lützelsachsen (DE)**
- **MARCO, Michael de**
  **Palo Alto, CA 94306 (US)**

(74) Vertreter: **BASF IP Association**
**BASF SE**
**G-FLP-C006**
**67056 Ludwigshafen (DE)**

(56) Entgegenhaltungen:
**WO-A-00/55245**
**WO-A-2005/054356**
**WO-A1-00/53664**
**WO-A1-2004/069936**
**WO-A1-2004/085496**
**WO-A1-2005/108472**
**WO-A1-2006/033477**
**WO-A1-2006/058682**
**WO-A1-2007/072969**
**WO-A2-2006/111402**
**WO-A2-2007/121937**
**JP-A- 2000 327 926**
**US-A- 5 002 986**
**US-A1- 2004 110 914**
**US-B1- 6 359 049**

- **Cutié S.S. et al: "The Effects of MEHQ on the Polymerization of Acrylic Acid in the Preparation of Superabsorbent Gels", J. Appl. Polym. Sci., vol. 64, 1997, pages 577-589,**
- **Buchholz F.L. Graham A.T.: "Modern Superabsorbent Polymer Technology", 1998, Wiley-VCH, New York, US**
- **Japanischer Standard Chemical Terms Dictionary mit Teilübersetzung D11a**
- **Ullmann's Encyclopedia of Industrial Chemistry (2006), Stichwort "Acrylis Acid and Derivatives". (=D2 der E1).**

EP 2 115 019 B2

- Ullmann’s Encyclopedia of Industrial Chemistry (2012), Stichwort "Superabsorbents". (=D4 der E1). Dieses Dokument trägt einen Copyright-Vermerk von 2012, fünf Jahre nach dem Prioritätsdatum des vorliegende Patents, und ist damit nicht Stand der Technik nach art. 54(2) oder (3) EPÜ. & Uns ist bekannt, dass zum gleichen Stichwort auch in der Auflage des "Ullmann’s" von 2003 existiert. Es kann jedoch nicht Aufgabe der Patentinhaberin oder des EPA sein, per inhaltsvergleich zu ermitteln, was sich in einem Artikel aus 2012 gegenüber Vorversionen geändert hat oder nicht. & Einer Neueinreichung steht der Einwand der Verspätung entgegen:Es ist definitiv nicht Sinn von R. 76 (2) c) EPÜ und Art. 114 (2) EPÜ, Einsprechenden die Einreichung Irgendeiner nachpublizierten Auflage eines Dokuments während der Einspruchsfrist und dessen Erzats durch eine andere, & womöglich vorpublizierte Auflage nach abl
- Ma S. et al: "Preparation and Properties of a Salt-Resistant Superabsorbent Polymer", Journal of Applied Polymer Science, vol. 93, 2004, pages 2532-2541,

## Beschreibung

Die vorliegende Erfindung betrifft ein Verfahren zur Herstellung wasserabsorbierender Polymerpartikel, wobei eine Monomerlösung oder-suspension, enthaltend mindestens eine ungesättigte Carbonsäure und mindestens einen Hydrochinonhalbether, polymerisiert wird, und die Polymerpartikel mit einem Salz eines dreiwertigen Metallkations einer Carbonsäure und/oder einem basischen Salz eines dreiwertigen Metallkations beschichtet werden, die durch das Verfahren herstellbaren wasserabsorbierbaren Polymerpartikel sowie deren Verwendung in Hygieneartikeln und Verpackungmaterialien.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie beispielsweise Flüssigkeitsleitfähigkeit (SFC) in der Windel und Absorption unter Druck (AUL0.7psi), werden wasserabsorbierende Polymerpartikel im allgemeinen nachvernetzt. Diese Nachvernetzung kann in wässriger Gelphase durchgeführt werden. Vorzugsweise werden aber gemahlene und abgesiebte Polymerpartikel (Grundpolymer) an der Oberfläche mit einem Nachvernetzer beschichtet, getrocknet und thermisch nachvernetzt. Dazu geeignete Vernetzer sind Verbindungen, die mindestens zwei Gruppen enthalten, die mit den Carboxylatgruppen des wasserabsorbierenden Polymeren kovalente Bindungen bilden können oder die mindestens zwei Carboxylgruppen oder andere funktionelle Gruppen mindestens zweier verschiedener Polymerketten des Grundpolymers kovalent oder ionisch miteinander vernetzen können.

Die Nachvernetzung wird in der Monographie "Modern Superabsorbent Polymer Technology", F.L. Buchholz und A.T. Graham, Wiley-VCH, 1998, Seiten 97 bis 103, beschrieben. Üblicherweise werden die wasserabsorbierenden Polymerpartikel mit dem Nachvernetzer beschichtet und thermisch nachvernetzt, wobei die Polymerpartikel mittels heißer Luft oder mittels Kontakttrocknung erwärmt und gleichzeitig getrocknet werden.

So beschreibt WO 2006/015729 A2 ein Verfahren zur Nachvernetzung, wobei Mischungen aus cyclischen Carbamaten und Diolen eingesetzt werden.

WO 2000/053664 A1 beschreibt die Herstellung von wasserabsorbierenden Polymerpartikeln mit hoher Absorption unter Druck (AUL0.7psi) und hoher Flüssigkeitsleitfähigkeit (SFC), wobei die Polymerpartikel mit einem organischem Nachvernetzer und einem ein- oder mehrwertigen Metallkation nachvernetzt werden.

Es ist aber auch möglich die Flüssigkeitsleitfähigkeit (SFC) durch Beschichtung mit wasserunlöslichen Metallphosphaten, beispielsweise Calciumphosphat, zu erhöhen. Ein derartiges Verfahren wird beispielsweise in WO 2006/015729 A2 beschrieben.

Für ultradünne Hygieneartikel werden wasserabsorbierende Polymerpartikel mit einem hohen Weißegrad benötigt. Die Herstellung solcher wasserabsorbierender Polymerpartikel mit hohem Weißegrad wird insbesondere in DE 10 2004 057 874 A1 beschrieben, welche ausdrücklich Bestandteil der vorliegenden Offenbarung ist. Bei ultradünnen Windeln oder Damenhygieneprodukten sind bereits geringe Farbvariationen durch die dünne Deckschicht zu erkennen, die von den Kunden nicht akzeptiert werden. Aber auch die Verwendung von Superabsorbern in dickeren Hygieneartikeln erfordert oftmals aus Akzeptanzgründen beim Kunden ein möglichst weißes Produkt, da Vergilbung oftmals mit schmutzig oder minderwertig assoziiert wird. Weiterhin kann es bei der Lagerung gewöhnlicher wasserabsorbierender Polymerpartikel bereits vor deren Verarbeitung, oder nach der Verarbeitung zum fertigen Hygieneartikel zu einer unerwünschten Vergilbung und Braunfärbung kommen. Dieser Effekt setzt die Lager- und Verkaufsfähigkeit der Hygieneprodukte herab und tritt insbesondere bei ungünstigen Lagerbedingungen, wie beispielsweise bei erhöhten Lagertemperaturen und hoher Luftfeuchte, auf. Insbesondere können sich so hergestellte Hygieneartikel noch vor dem Verkauf im Einzelhandel oder vor dem Verbrauch beim Endverbraucher verfärben und zu Reklamationen führen. Eine denkbare separate Verpackung jedes einzelnen Hygieneartikel ist aber mit hohen Kosten verbunden.

Weiterhin dürfen die wasserabsorbierenden Polymerpartikel keine unangenehmen Gerüche abgeben, insbesondere bei Flüssigkeitsbeladung während der Verwendung des Hygieneartikels.

Es bestand daher die Aufgabe ein verbessertes Verfahren zur Herstellung wasserabsorbierender Polymerpartikel bereitzustellen, bei dem Polymerpartikel mit einem hohen Weißegrad erzeugt werden, deren Weißegrad auch bei längerer Lagerung unter ungünstigen klimatischen Bedingungen weitgehend erhalten bleibt.

Eine weitere Aufgabe war die Bereitstellung eines Verfahrens zur Herstellung wasserabsorbierender Polymerpartikel, wobei Polymerpartikel erzeugt werden, die frei von wahrnehmbaren Gerüchen sind, insbesondere bei Flüssigkeitsbeladung.

Weiterhin bestand die Aufgabe wasserabsorbierende Polymerpartikel mit einem hohen Weißegrad zur Verwendung in Hygieneartikeln bereitzustellen.

Weiterhin bestand die Aufgabe wasserabsorbierende Polymerpartikel mit einem hohen Weißegrad, hoher Flüssigkeitsleitfähigkeit, hohem Absorptionsvermögen und guter Farbstabilität nach längerer Lagerung zur Verwendung in ultradünnen Hygieneartikeln bereitzustellen.

Gelöst wurde die Aufgabe der vorliegenden Erfindung durch ein Verfahren zur Herstellung wasserabsorbierender Polymerpartikel, wobei eine Monomerlösung oder -suspension, enthaltend

a) mindestens eine ungesättigte Carbonsäure, wobei die ungesättigte Carbonsäure zumindest teilweise neutralisiert sein kann, und

b) mindestens einen Hydrochinonhalbether

polymerisiert wird und die Polymerpartikel mit mindestens einem Salz eines dreiwertigen Metallkations beschichtet werden, dadurch gekennzeichnet, dass das Salz des dreiwertigen Metallkations das Salz einer Carbonsäure und/oder ein basisches Salz ist und dadurch gekennzeichnet, dass die Polymerpartikel mit mindestens einem basischen Salz eines zweiwertigen Metallkations beschichtet werden.

Die ungesättigte Carbonsäure a) ist vorzugsweise zu 25 bis 95 mol-%, besonders bevorzugt zu 27 bis 80 mol-%, ganz besonders bevorzugt zu 27 bis 30 mol-% oder 40 bis 75 mol-%, neutralisiert.

Die Monomerlösung enthält, bezogen auf die ungesättigte Carbonsäure a), vorzugsweise von 0,001 bis 0,016 Gew.-%, besonders bevorzugt von 0,003 bis 0,013 Gew.-%, ganz besonders bevorzugt von 0,005 bis 0,007 Gew.-%, Hydrochinonhalbether b). Der bevorzugte Hydrochinonhalbether ist Hydrochinonmonomethylether.

Das dreiwertige Metallkation ist vorzugsweise ein Metallkation der dritten Hauptgruppe, der dritten Nebengruppe oder der Lanthanidengruppe des Periodensystems der Elemente, besonders bevorzugt Aluminium, Scandium, Yttrium, Lanthan oder Cer, ganz besonders bevorzugt Aluminium.

Die Salze der dreiwertigen Metallkationen sind vorzugsweise Salze von Carbonsäuren und/oder basische Salze, wie Acetate, Propionate, Tartrate, Maleate, Citrate, Laktate und/oder Hydroxide, besonders bevorzugt Salze von 2-Hydroxycarbonsäuren, wie Citrate und/oder Laktate, ganz besonders bevorzugt Laktate.

Hydroxide dreiwertiger Metallkationen sind insbesondere wasserlösliche und wasserdispergierbare Alkali- und Erdalkalimetallaluminate und deren Hydrate, vorzugsweise Natriumaluminat und dessen Hydrate.

Geeignete Salze dreiwertiger Metallkationen sind beispielsweise Aluminiumacetat, A-luminiumpropionat, Aluminiumcitrat, Aluminiumlaktat und Natriumaluminat.

Die Menge an Salz des dreiwertigen Metallkations, bezogen auf die Polymerpartikel, beträgt typischerweise von 0,001 bis 5 Gew.-%, vorzugsweise von 0,01 bis 2,5 Gew.-%, bevorzugt 0,1 bis 1,5 Gew.-%, besonders bevorzugt von 0,1 bis 1 Gew.-%, ganz besonders bevorzugt von 0,4 bis 0,7 Gew.-%.

Das Salz des dreiwertigen Metallkations kann als Lösung oder Suspension eingesetzt werden.

Basische Salze sind Salze, die geeignet sind den pH-Wert einer sauren wäßrigen Lösung zu erhöhen, vorzugsweise einer 0,1 N Salzsäure. Basische Salze sind üblicherweise Salze einer starken Base mit einer schwachen Säure.

Das zweiwertige Metallkation ist vorzugsweise ein Metallkation der zweiten Hauptgruppe des Periodensystems der Elemente, besonders bevorzugt Calcium und/oder Magnesium, ganz besonders bevorzugt Calcium.

Die Salze der zweiwertigen Metallkationen sind vorzugsweise Salze schwacher anorganischer Säuren, schwacher organischer Säuren und/oder Salze von Aminosäuren, besonders bevorzugt Hydroxide, Hydrogencarbonate, Carbonate, Acetate, Propionate, Citrate, Glukonate, Laktate, Tartrate, Maleate und/oder Fumarate, ganz besonders bevorzugt Hydroxide, Hydrogencarbonate, Carbonate, Propionate und/oder Laktate.

Geeignete Salze zweiwertiger Metallkationen sind beispielsweise Calciumhydroxid, Magnesiumhydroxid, Zinkoxid, Calciumhydrogencarbonat, Magnesiumhydrogencarbonat, Calciumacetat, Magnesiumacetat, Calciumpropionat, Magnesiumpropionat, Calciumcarbonat und Magnesiumcarbonat.

Die Menge an basischem Salz des zweiwertigen Metallkations, bezogen auf die Polymerpartikel, beträgt typischerweise von 0,001 bis 5 Gew.-%, vorzugsweise von 0,01 bis 2,5 Gew.-%, bevorzugt 0,1 bis 1,5 Gew.-%, besonders bevorzugt von 0,1 bis 1 Gew.-%, ganz besonders bevorzugt von 0,4 bis 0,7 Gew.-%.

Das basische Salz des zweiwertigen Metallkations kann als Lösung oder Suspension eingesetzt werden. Beispiele hierfür sind Calciumlaktat-Lösungen oder Calciumhydroxid-Suspensionen.

Die eingesetzten Salze des zwei und/ dreiwertigen Metallkations können weitere Nebenbestandteile wie noch unneutralisierte Carbonsäure und/oder Alkalisalze der neutralisierten Carbonsäure enthalten. Bevorzugte Alkalisalze sind die des Natriums, Kaliums und des Ammoniums. Sie werden typischerweise als wässerige Lösung eingesetzt welche durch Auflösen der festen Salze in Wasser gewonnen wird, oder bevorzugt direkt als solche erzeugt wird, wodurch gegebenenfalls Trocknungs- und Reinigungsschritte vermieden werden.

In einer besonders bevorzugten Ausführungsform der vorliegenden Erfindung werden die wasserabsorbierenden Polymerpartikel thermisch nachvernetzt.

Dabei werden die wasserabsorbierenden Polymerpartikel vorzugsweise vor der thermischen Nachvernetzung

mit dem dreiwertigen Metallkation und mit dem basischen Salz des zweiwertigen Metallkations beschichtet.

Der Gesamtpartialdruck eines oder mehrerer oxidierender Gase in der die Polymerpartikel während der thermischen Nachvernetzung überlagernden Atmosphäre beträgt vorzugsweise weniger als 140 mbar, bevorzugt weniger als 100 mbar, besonders bevorzugt weniger als 50 mbar, ganz besonders bevorzugt weniger als 10 mbar.

Oxidierende Gase sind Stoffe, die bei 23 °C einen Dampfdruck von mindestens 1013 mbar aufweisen und in Verbrennungsvorgängen als Oxidationsmittel wirken, beispielsweise Sauerstoff, Stickoxid und Stickstoffdioxid, insbesondere Sauerstoff.

Der Sauerstoffpartialdruck während der thermischen Nachvernetzung in der die wasserabsorbierenden Polymerpartikel überlagernden Atmosphäre beträgt vorzugsweise weniger als 140 mbar, bevorzugt weniger 100 mbar, besonders bevorzugt weniger als 50 mbar, ganz besonders bevorzugt weniger als 10 mbar.

Wird die thermische Nachvernetzung bei Umgebungsdruck, d. h. bei einem Gesamtdruck um 1013 mbar, durchgeführt, so wird der Gesamtpartialdruck der oxidierenden Gase über deren Volumenanteil festgelegt. Der Anteil der oxidierenden Gase beträgt dabei vorzugsweise weniger als 14 Vol.-%, bevorzugt weniger als 10 Vol.-%, besonders bevorzugt weniger als 5 Vol.-%, ganz besonders bevorzugt weniger als 1 Vol.-%.

Vorzugsweise wird die thermische Nachvernetzung bei vermindertem Druck durchgeführt, d. h. bei einem Gesamtdruck von weniger als 1.013 mbar. Der Gesamtdruck beträgt typischerweise weniger als 670 mbar, vorzugsweise weniger als 480 mbar, besonders bevorzugt weniger als 300 mbar, ganz besonders bevorzugt weniger als 200 mbar. Werden Trocknung und Nachvernetzung unter Luft mit einem Sauerstoffgehalt von 20,8 Vol.-% durchgeführt, so betragen die zu den obengenannten Gesamtdrücken korrespondierenden Sauerstoffpartialdrücke 139 mbar (670 mbar), 100 mbar (480 mbar), 62 mbar (300 mbar) und 42 mbar (200 mbar), wobei die jeweiligen Gesamtdrücke in den Klammern stehen.

Vorteilhaft werden grobkörnige Polymerpartikel vor der thermischen Nachvernetzung abgetrennt. Beispielsweise mittels eines Siebes mit einer Maschenweite von vorzugsweise 700 $\mu$m oder weniger, besonders bevorzugt 650 $\mu$m oder weniger, ganz besonders bevorzugt 600 $\mu$m oder weniger.

Bevorzugte Nachvernetzer sind 1,3-Diole, wie 1,3-Propandiol, 1,3-Butandiol und 2-Methyl-1,3-proprandiol, und/oder cyclische Carbamate, wie 2-Oxazolidon, und N-(2-Hydroxyethyl)-2-oxazolidon.

Bei Verwendung von Monomerlösungen, die sowohl Hydrochinonmonomethylether als auch Natriumperoxodisulfat enthalten, werden wasserabsorbierende Polymerpartikel mit ausgeprägter Tendenz zu Verfärbungen erhalten. Eine Möglichkeit zur Herstellung weißer wasserabsorbierender Polymerpartikel war daher die Abtrennung von Hydrochinonmonomethylether vor der Polymerisation. Es war aber auch möglich Natriumperoxodisulfat durch andere Polymerisationsinitiatoren zu ersetzen.

Der vorliegenden Erfindung liegt die Erkenntnis zugrunde, dass auch Salze starker Säure, wie Aluminiumsulfat, bei der Lagerung unter klimatisch ungünstigen Bedingungen zu Verfärbungen führen können. Es wurde nun gefunden, dass Salze schwacher Säuren diese unerwünschte Eigenschaft nicht aufweisen.

Weiterhin lassen sich diese Verfärbungen durch Beschichtung der Polymerpartikel mit basischen Salzen zweiwertiger Metallkationen verhindern.

Die Langzeitstabilität gegen Verfärbungen kann weiter erhöht werden, wenn größere Polymerpartikel vor der thermischen Nachvernetzung abgetrennt werden.

Weiterhin werden durch Verwendung dreiwertiger Metallsalze schwacher Säuren statt Aluminiumsulfat wasserabsorbierende Polymerpartikel mit einem verbesserten Eigenschaftsprofil, d.h. höherer Flüssigkeitsleitfähigkeit (SFC) und höherer Absorption unter Druck (AUL0.7psi), erhalten.

Die vernetzten, wasserabsorbierenden Polymerpartikel werden beispielsweise in der Monographie "Modern Superabsorbent Polymer Technology", F.L. Buchholz und A.T. Graham, Wiley-VCH, 1998, oder in Ullmann's Encyclopedia of Industrial Chemistry, 6. Auflage, Band 35, Seiten 73 bis 103, beschrieben.

Die wasserabsorbierenden Polymerpartikel werden durch Polymerisation einer Monomerlösung oder -suspension, enthaltend

a) mindestens eine ungesättigte Carbonsäure,
b) mindestens einen Hydrochinonhalbether,
c) optional ein oder mehrere anorganische Peroxide,
d) optional einen oder mehrere Vernetzer,
e) optional ein oder mehrere mit a) copolymerisierbare ethylenisch und/oder allylisch ungesättigte Monomere und
f) optional ein oder mehrere wasserlösliche Polymeren, auf die die Monomere a), d) und e) zumindest teilweise aufgepfropft werden können,

erhalten.

Geeignete ungesättigte Carbonsäuren a) sind beispielsweise ethylenisch ungesättigte Carbonsäuren, wie Acrylsäure, Methacrylsäure, Maleinsäure, Fumarsäure und Itaconsäure. Besonders bevorzugte ungesättigte Carbonsäuren a) sind Acrylsäure und Methacrylsäure. Ganz besonders bevorzugt ist Acrylsäure.

Hydrochinonhalbether b) sind Hydrochinone, die an genau einer Hydroxygruppe verethert sind. Geeignete Hydrochinonhalbether b) sind Hydrochinonmonoalkoxyether, wie Hydrochinonmonomethylether und Hydrochinonmonoethylether, vorzugsweise Hydrochinonmonomethylether, und/oder Tocopherole, wie alpha-Tocopherol, racemisches alpha-Tocopherol und RRR-alpha-Tocopherol, vorzugsweise racemisches alpha-Tocopherol und RRR-alpha-Tocopherol.

Die Monomerlösung enthält vorzugsweise höchstens 0,016 Gew.-%, besonders bevorzugt höchstens 0,013 Gew.-%, ganz besonders bevorzugt höchstens 0,007 Gew.-%, vorzugsweise mindestens 0,001 Gew.-%, besonders bevorzugt mindestens 0,003 Gew.-%, ganz besonders bevorzugt mindestens 0,005 Gew.-%, Hydrochinonhalbether, jeweils bezogen auf ungesättigte Carbonsäure a), wobei ungesättigte Carbonsäuresalze rechnerisch als ungesättigte Carbonsäure a) mit berücksichtigt werden. Beispielsweise kann zur Herstellung der Monomerlösung eine Acrylsäure mit einem entsprechenden Gehalt an Hydrochinonhalbether verwendet werden.

Das anorganische Peroxid c) ist beilspielsweise Wasserstoffperoxid, ein Persulfat, wie Natriumperoxodisulfat, und/oder ein Perphosphat, wie Natriumperoxodiphosphat, vorzugsweise ein Salz, besonders bevorzugt ein Persulfat oder ein Perphosphat, ganz besonders bevorzugt ein Persulfat, beispielsweise Natriumperoxodisulfat.

Im erfindungsgemäßen Verfahren wird vorzugsweise ein Initiatorsystem verwendet, welches Peroxodisulfat und/oder Peroxodiphosphat enthält.

Zum Starten der Reaktion wird im erfindungsgemäßen Verfahren bevorzugt ein Initiatorsystem enthaltend Peroxodisulfat, beispielsweise Natriumperoxodisulfat, Kaliumperoxodisulfat und/oder Ammoniumperoxodisulfat, Ascorbinsäure und Wasserstoffperoxid verwendet. Die beiden Komponenten Ascorbinsäure und Wasserstoffperoxid können durch jeden anderen dem Fachmann bekannten Initiator ersetzt oder ergänzt werden. Solche Substitute umfassen andere Reduktions- und Oxidationsmittel, und auch die bekannten Azo- und Photoinitiatoren sowie katalytisch wirksame Metallkationen wie beispielsweise Eisenkationen.

Die wasserabsorbierenden Polymere sind vorzugsweise vernetzt, d. h. die Polymerisation wird in Gegenwart von Verbindungen mit mindestens zwei polymerisierbaren Gruppen, die in das Polymernetzwerk radikalisch einpolymerisiert werden können, durchgeführt. Geeignete Vernetzer d) sind beispielsweise Ethylenglykoldimethacrylat, Diethylenglykoldiacrylat, Allylmethacrylat, Trimethylolpropantriacrylat, Triallylamin, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/32962 A2 beschrieben.

Geeignete Vernetzer d) sind insbesondere N,N' -Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP 0 343 427 A2 beschrieben sind. Weiterhin geeignete Vernetzer d) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufinreist.

Besonders vorteilhafte Vernetzer d) sind jedoch Di- und Triacrylate des 3- bis 20-fach ethoxylierten Glyzerins, des 3- bis 20-fach ethoxylierten Trimethylolpropans, des 3- bis 20-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des mindestens 40-fach ethoxylierten Glyzerins, Trimethylolethans oder Trimethylolpropans.

Ganz besonders bevorzugte Vernetzer d) sind die mit Acrylsäure oder Methacrylsäure zu Di- oder Triacrylaten veresterten mehrfach ethoxylierten und/oder propoxylierten Glyzerine wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins. Diese zeichnen sich durch besonders niedrige Restgehalte (typischerweise unter 0,0010 Gew.-%) in den wasserabsorbierenden Polymerpartikeln aus und die wässrigen Extrakte der damit hergestellten wasserabsorbierenden Polymerpartikeln weisen eine fast unveränderte Oberflächenspannung (typischerweise mindestens 0,068 N/m) im Vergleich zu Wasser gleicher Temperatur auf.

Mit den ungesättigten Carbonsäuren a) copolymerisierbare ethylenisch ungesättigte Monomere e) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentyl methacrylat.

Als wasserlösliche Polymere f) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die Herstellung eines geeigneten Grundpolymers sowie weitere geeignete hydrophile ethylenisch ungesättigte Carbonsäuren a) werden in DE 199 41 423 A1, EP 0 686 650 A1, WO 2001/45758 A1 und WO 2003/104300 A1 beschrieben.

Die Umsetzung wird vorzugsweise in einem Kneter, wie beispielsweise in WO 2001/38402 A1 beschrieben, oder auf einem Bandreaktor, wie beispielsweise in EP 0 955 086 A2 beschrieben, durchgeführt.

Die Säuregruppen der erhaltenen Hydrogele sind üblichenweise teilweise neutralisiert, vorzugsweise zu 25 bis 95 mol-%, bevorzugt zu 27 bis 80 mol-%, besonders bevorzugt zu 27 bis 30 mol-% oder 40 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Optional oder im Gemisch mit den genannten Alkaliverbindungen können zur Neutralisation auch die entsprechenden Erdalkaliverbindungen und hier bevorzugt die Verbindungen des Magnesiums und des Calciums verwendet werden. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Die Neutralisation kann nach der Polymerisation auf der Stufe des Hydrogels durchgeführt werden. Es ist aber auch möglich bis zu 40 mol-%, vorzugsweise 10 bis 30 mol-%, besonders bevorzugt 15 bis 25 mol-%, der Säureguppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Hydrogels eingestellt wird. Die Monomerlösung kann durch Einmischen des Neutralisationsmittels neutralisiert werden. Das Hydrogel kann mechanisch zerkleinert werden, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden. Die Neutralisation der Monomerlösung direkt auf den Endneutralisationsgrad ist bevorzugt.

In einer besonders bevorzugten Ausführungsform der Erfindung wird der Neutralisations-grad mittels 50gew.-%iger Natronlauge der Qualität "Membrane grade" auf 65 bis 72 mol-% eingestellt.

Das neutralisierte Hydrogel wird dann mit einem Band- oder Walzentrockner getrocknet bis der Restfeuchtegehalt vorzugsweise unter 15 Gew.-%, insbesondere unter 10 Gew.-% liegt, wobei der Wassergehalt gemäß der von der EDANA (European Disposables and Nonwovens Association) empfohlenen Testmethode Nr. WSP 230.2-05 "Moisture content" bestimmt wird. Wahlweise kann zur Trocknung aber auch ein Wirbelbetttrockner oder ein beheizter Pflugscharmischer verwendet werden. Um besonders weiße Produkte zu erhalten, ist es vorteilhaft bei der Trocknung dieses Gels einen schnellen Abtransport des verdampfenden Wassers sicherzustellen. Dazu ist die Trocknertemperatur zu optimieren, die Luftzu- und -abführung muss kontrolliert erfolgen, und es ist in jedem Fall auf ausreichende Belüftung zu achten. Die Trocknung ist naturgemäß um so einfacher und das Produkt um so weißer, wenn der Feststoffgehalt des Gels möglichst hoch ist. Bevorzugt liegt der Feststoffgehalt des Gels vor der Trocknung daher zwischen 30 und 80 Gew.-%. Besonders vorteilhaft ist die Belüftung des Trockners mit Stickstoff oder einem anderen nicht-oxidierenden Inertgas. Wahlweise kann aber auch einfach nur der Partialdruck des Sauerstoffs während der Trocknung abgesenkt werden, um oxidative Vergilbungsvorgänge zu verhindern. Im Regelfall führt aber auch eine ausreichende Belüftung und Abführung des Wasserdampfes zu einem noch akzeptablen Produkt. Vorteilhaft hinsichtlich Farbe und Produktqualität ist in der Regel eine möglichst kurze Trocknungszeit.

Eine weitere wichtige Funktion der Trocknung des Gels ist die hier noch stattfindende Verringerung des Restmonomerengehaltes in den Polymerpartikeln. Bei der Trocknung zerfallen nämlich eventuell noch vorhandene Reste der Initiatoren und führen zu einer Einpolymerisation von noch vorhandenen Restmonomeren. Außerdem reißen die verdampfenden Wassermengen noch vorhandene freie wasserdampfflüchtige Monomere, wie beispielsweise Acrylsäure mit, und verringern so ebenfalls den Restmonomerengehalt in den Polymerpartikeln.

Das getrocknete Hydrogel wird hiernach gemahlen und gesiebt, wobei zur Mahlung üblicherweise ein- oder mehrstufige Walzenstühle, bevorzugt zwei- oder dreistufige Walzenstühle, Stiftmühlen, Hammermühlen oder Schwingmühlen eingesetzt werden können.

Die Partikelgrößen der gemahlenen Polymerpartikel des getrockneten Hydrogels liegen nach Absiebung typischerweise im Bereich von 0 bis 1000 μm, vorzugsweise im Bereich von 150 bis 850 μm, besonders bevorzugt im Bereich 200 bis 800 μm, ganz besonders bevorzugt im Bereich 200 bis 700 μm.

Vorzugsweise weisen weniger als 2 Gew.-%, besonders bevorzugt weniger als 1,5 Gew.-%, ganz besonders bevorzugt weniger als 1 Gew.-%, der Polymerpartikel eine Partikelgröße von über 850 μm auf.

Vorzugsweise weisen weniger als 5 Gew.-%, bevorzugt weniger als 3 Gew.-%, besonders bevorzugt weniger als 1 Gew.-%, ganz besonders bevorzugt weniger als 0,3 Gew.-% der Polymerpartikel eine Partikelgröße von unter 150 μm auf.

Vorzugsweise weisen mindestens 90 Gew.-%, bevorzugt mindestens 95 Gew.-%, besonders bevorzugt mindestens 98 Gew.-%, ganz besonders bevorzugt mindestens 99 Gew.-%, der Polymerpartikel eine Partikelgröße von 150 bis 850 μm auf.

In einer bevorzugten Ausführung weisen mindestens 90 Gew.-%, bevorzugt mindestens 95 Gew.-%, besonders bevorzugt mindestens 98 Gew.-%, ganz besonders bevorzugt mindestens 99 Gew.-%, der Polymerpartikel eine Partikelgröße von 150 bis 700 μm auf.

In einer besonders bevorzugten Ausführung weisen mindestens 90 Gew.-%, bevorzugt mindestens 95 Gew.-%, besonders bevorzugt mindestens 98 Gew.-%, ganz besonders bevorzugt mindestens 99 Gew.-%, der Polymerpartikel eine Partikelgröße von 150 bis 600 μm auf.

In einer weiteren besonders bevorzugten Ausführung weisen mindestens 85 Gew.-%, bevorzugt mindestens 90 Gew.-%, besonders bevorzugt mindestens 98 Gew.-%, ganz besonders bevorzugt mindestens 99 Gew.-%, der Polymerpartikel eine Partikelgröße von 200 bis 500 μm auf.

Es können gemäß dem erfindungsgemäßen Verfahren vorteilhaft auch sphärische Partikel durch Suspensions-, Sprüh- oder Tropfenpolymerisationsverfahren hergestellt werden. Besonders geeignet sind Partikel mit enger Korngrößenverteilung sowie irreguläre Partikel entstanden durch Agglomeration sphärischer Partikel. Es ist im erfindungsgemäßen Verfahren selbstverständlich möglich auch irreguläre Partikel oder reguläre nicht-sphärische Partikel aus solchen Polymerisationen herzustellen.

In einer Ausführungsform des erfindungsgemäßen Verfahrens wird das Grundpolymer mit mindestens einem Salz eines dreiwertigen Metallkations einer Carbonsäure und mindestens einem basischen Salz eines zweiwertigen Metallkations beschichtet. Das Salz des dreiwertigen Metallkations ist vorzugsweise wasserlöslich.

Vorzugsweise wird eine wässrige Lösung enthaltend Aluminiumkationen auf die wasserabsorbierenden Polymerpartikel aufgesprüht.

In einer weiteren bevorzugten Ausführungsform des erfindungsgemäßen Verfahrens wird das Grundpolymer mit mindestens einem dreiwertigen Metallkation ausgewählt aus den wasserlöslichen Salzen der Elemente der dritten Hauptgruppe, der dritten Nebengruppe oder der Lanthanidengruppe des Periodensystems der Elemente, bevorzugt Aluminium, Scandium, Yttrium, Lanthan und/oder Cer, und mindestens einem basischen Salz eines zweiwertigen Metallkations beschichtet.

In einer ganz besonders bevorzugten Ausführungsform des erfindungsgemäßen Verfahrens wird das Grundpolymer mit mindestens einem wasserlöslichen Salz des Aluminiums und mindestens einem wasserlöslichen basischen Salz des Calciums beschichtet.

Vorzugsweise wird eine wässrige Lösung, enthaltend Aluminiumkationen und Calcium und/oder Magnesiumkationen, auf die wasserabsorbierenden Polymerpartikel aufgesprüht.

Wasserlösliche Salze im erfindungsgemäßen Sinn, sind Salze der zwei- und dreiwertigen Metallkationen, welche bei 20 °C eine Wasserlöslichkeit von mindestens 0,5 g Salz pro Liter Wasser, vorzugsweise mindestens 1 g Salz pro l Wasser, bevorzugt mindestens 10 g Salz pro l Wasser, besonders bevorzugt mindestens 100 g Salz pro l Wasser, ganz besonders bevorzugt mindestens 200 g Salz pro l Wasser, aufweisen. Im erfindungsgemäßen Sinn einsetzbar sind auch solche Salze, die diese Mindestlöslichkeit bei der Aufsprühtemperatur der Sprühlösung aufweisen.

Wenn diese Wasserlöslichkeit nicht ausreicht eine Sprühlösung der gewünschten Konzentration herzustellen, dann können auch Dispersionen des festen Salzes in seiner gesättigten wässerigen Lösung eingesetzt werden.

Wenn die Lösung des dreiwertigen Metallkations nicht mit der Lösung des zweiwertigen Metallkations ohne Ausfällung mischbar ist, so können die Lösungen separat nacheinander oder zeitgleich aus zwei Düsen aufgesprüht werden.

Besonders bevorzugt sind solche Salze, bei denen sich die wässrigen Lösungen, welche das dreiwertige Metallkation und das zweiwertige Metallkation enthalten, beliebig mischen lassen.

Im erfindungsgemäßen Sinn geeignete Salze drei- und zweiwertiger Metallkationen weisen vorzugsweise Anionen auf, welche sich von schwachen anorganischen Säuren oder schwachen organischen Carbonsäuren oder Aminosäuren ableiten. Die zugrunde liegenden Säuren der Anionen sind bei den erfindungsgemäßen Salzen entweder leicht flüchtig wie beispielsweise Kohlensäure, schweflige Säure, Wasser oder Essigsäure, oder thermisch zersetzbar wie beispielsweise Milchsäure oder Weinsäure, und sie können auch thermisch stabil unter den Trocknungsbedingungen sein.

Geeignet im erfindungsgemäßen Verfahren sind beispielsweise Aluminiumacetat, Aluminiumlaktat, Aluminiumpropionat, Aluminiumcitrat, Natriumaluminat, säurelösliches Aluminiumhydroxid, Calciumhydroxid, Calciumhydrogencarbonat, Calciumacetat, Calciumpropionat, Calciumcitrat, Calciumglukonat, Calciumlaktat, Calciumtartrat, Calciummaleat und Calciumfumarat. Die diesen entsprechenden Salze mit anderen erfindungsgemäßen Metallkationen können ebenfalls eingesetzt werden.

Besonders bevorzugt im erfindungsgemäßen Verfahren sind Aluminiumlaktat, Calciumhydroxid, Calciumhydrogencarbonat, Calciumpropionat und Calciumlaktat.

Vorteilhaft können auch die Hydrate der obengenannten Salze eingesetzt werden. Die Hydrate, beispielsweise das Pentahydrat des Aluminiumlaktats, lösen sich schneller in Wasser als die wasserfreien Salze.

Üblicherweise werden die Salze mit einer Wassermenge von nicht mehr als 15 Gew.-% vorzugsweise von nicht mehr als 8 Gew.-%, besonders bevorzugt von nicht mehr als 5 Gew.-%, ganz besonders bevorzugt von nicht mehr als 2 Gew.-% bezogen auf die eingesetzten Polymerpartikel aufgesprüht. Im erfindungsgemäßen Verfahren werden vorteilhaft wasserlösliche Salze eingesetzt.

In einer weiteren besonderen Ausführungsform ist es jedoch auch möglich ein Salz eines dreiwertigen Metallkations zusammen mit in Wasser fein dispergiertem Calciumcarbonat aufzusprühen. Das Salz des dreiwertigen Metallkation ist hierbei vorzugsweise wasserlöslich und ist bevorzugt ein Aluminiumsalz, besonders bevorzugt Aluminiumlaktat. In diesem Fall weist das Calciumcarbonat Korngrößen von vorzugsweise weniger als 50 $\mu$m, bevorzugt weniger als 30 $\mu$m, besonders bevorzugt weniger als 10 $\mu$m, ganz besonders bevorzugt von 0,01 bis 5 $\mu$m, auf.

Die Grundpolymere werden vorzugsweise nachvernetzt. Hierzu geeignete Nachvernetzer sind Verbindungen, die mindestens zwei Gruppen enthalten, die mit den Carboxylatgruppen der Polymere kovalente Bindungen bilden können. Geeignete Verbindungen sind beispielsweise Alkoxysilylverbindungen, Polyaziridine, Polyamine, Polyamidoamine, Di- oder Polyglycidylverbindungen, wie in EP 0 083 022 A2, EP 0 543 303 A1 und EP 0 937 736 A2 beschrieben, mehrwertige Alkohole, wie in DE 33 14 019 A1, DE 35 23 617 A1 und EP 0 450 922 A2 beschrieben, oder $\beta$-Hydroxyalkylamide, wie in DE 102 04 938 A1 und US 6,239,230 beschrieben. Geeignet sind ferner Verbindungen mit gemischter Funktionalität, wie Glycidol, 3-Ethyl-3-oxetanmethanol (Trimethylolpropanoxetan), wie in EP 1 199 327 A2 beschrieben, Aminoethanol, Diethanolamin, Triethanolamin oder Verbindungen, die nach der ersten Reaktion eine weitere Funktionalität ausbilden, wie Ethylenoxid, Propylenoxid, Isobutylenoxid, Aziridin, Azetidin oder Oxetan.

Des weiteren sind in DE 40 20 780 C1 cyclische Carbonate, in DE 198 07 502 A1 2-Oxazolidon und dessen Derivate, wie N-(2-Hydroxyethyl)-2-oxazolidon, in DE 198 07 992 C1 Bis- und Poly-2-oxazolidone, in DE 198 54 573 A1 2-Oxotetrahydro-1,3-oxazin und dessen Derivate, in DE 198 54 574 A1 N-Acyl-2-Oxazolidone, in DE 102 04 937 A1 cyclische Harnstoffe, in DE 103 34 584 A1 bicyclische Amidacetale, in EP 1 199 327 A2 Oxetane und cyclische Harnstoffe und in WO 2003/031482 A1 Morpholin-2,3-dion und dessen Derivate als geeignete Nachvernetzer beschrieben.

Die Nachvernetzung wird üblicherweise so durchgeführt, dass eine Lösung des Nachvernetzers auf das Hydrogel oder die trockenen Grundpolymerpartikel aufgesprüht wird. Im Anschluss an das Aufsprühen wird thermisch getrocknet, wobei die Nachvernetzungsreaktion sowohl vor als auch während der Trocknung stattfinden kann.

Die Lösung, enthaltend das mindestens eine dreiwertige Metallkation, kann vor, während oder nach der Nachvernetzung getrennt dosiert und aufgesprüht oder direkt der Nachvernetzerlösung zugesetzt werden. Vorzugsweise wird das mindestens eine dreiwertige Metallkation der Nachvernetzerlösung einfach direkt zugesetzt. Wenn das mindestens eine dreiwertige Metallkation vor oder nach der Nachvernetzung getrennt aufgetragen wird kann jede dem Fachmann bekannte Beschichtungsapparatur und jeder Mischer verwendet werden.

Die Lösung, enthaltend das mindestens eine dreiwertige Metallkation und das indestens eine zweiwertige Metallkation, kann vor, während oder nach der Nachvernetzung getrennt dosiert und aufgesprüht oder direkt der Nachvernetzerlösung zugesetzt werden. Vorzugsweise werden das drei- und das zweiwertige Metallkation der Nachvernetzerlösung einfach direkt zugesetzt. Wenn die Lösung enthaltend das mindestens eine dreiwertige Metallkation und das mindestens eine zweiwertige Metallkation vor oder nach der Nachvernetzung getrennt aufgetragen wird kann jede dem Fachmann bekannte Beschichtungsapparatur und jeder Mischer verwendet werden. Die beiden Metallkationen, vorzugsweise Aluminium- und Calciumkationen, können auch aus getrennten Lösungen zeitlich parallel dosiert, zeitlich überlappend dosiert oder zeitlich nacheinander aufgetragen werden.

Besonders bevorzugt sind solche Salze, bei denen sich die wässrigen Lösungen, welche das dreiwertige Metallkation und das zweiwertige Metallkation enthalten, beliebig mischen lassen. Ganz besonders bevorzugt sind Gemische solcher Lösungen, die sich direkt mit der Nachvernetzungslösung mischen lassen, ohne dass es hierbei zu Ausfällungen kommt.

Vorzugsweise werden die wasserlöslichen Salze direkt in der wässrigen Nachvernetzerlösung, gegebenenfalls unter Zusatz von Wasser, aufgelöst, oder die wasserlöslichen Salze werden in Wasser gelöst oder gegebenenfalls in ihrer gesättigten Lösung dispergiert.

Bevorzugte Nachvernetzer sind Amidacetale oder Carbamate der allgemeinen Formel (I)

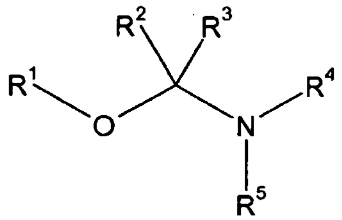

(I)

worin

$R^1$ $C_1$-$C_{12}$-Alkyl, $C_2$-$C_{12}$-Hydroxyalkyl, $C_2$-$C_{12}$-Alkenyl oder $C_6$-$C_{12}$-Aryl,

$R^2$ X oder $OR^6$

$R^3$ Wasserstoff, $C_1$-$C_{12}$-Alkyl, $C_2$-$C_{12}$-Hydroxyalkyl, $C_2$-$C_{12}$-Alkenyl oder $C_6$-$C_{12}$-Aryl, oder X,

$R^4$ $C_1$-$C_{12}$-Alkyl, $C_2$-$C_{12}$-Hydroxyalkyl, $C_2$-$C_{12}$-Alkenyl oder $C_6$-$C_{12}$-Aryl,

$R^5$ Wasserstoff, $C_1$-$C_{12}$-Alkyl, $C_2$-$C_{12}$-Hydroxyalkyl, $C_2$-$C_{12}$-Alkenyl, $C_1$-$C_{12}$-Acyl oder $C_6$-$C_{12}$-Aryl,

$R^6$ $C_1$-$C_{12}$-Alkyl, $C_2$-$C_{12}$-Hydroxyalkyl, $C_2$-$C_{12}$-Alkenyl oder $C_6$-$C_{12}$-Aryl und

X ein für die Reste $R^2$ und $R^3$ gemeinsamer Carbonylsauerstoff

bedeuten, wobei $R^1$ und $R^4$ und/oder $R^5$ und $R^6$ ein verbrücktes $C_2$-$C_6$-Alkandiyl sein können, und wobei die obengenannte Reste $R^1$ bis $R^6$ noch insgesamt über ein bis zwei freie Valenzen verfügen können und mit diesen freien Valenzen mit mindestens einem geeigneten Grundkörper verbunden sein können,
oder mehrwertige Alkohole, wobei der mehrwertige Alkohol vorzugsweise ein Molekulargewicht von weniger als 100 g/mol, bevorzugt von weniger als 90 g/mol, besonders bevorzugt von weniger als 80 g/mol, ganz besonders bevorzugt von weniger als 70 g/mol, pro Hydroxygruppe sowie keine vincinalen, geminalen, sekundären oder tertiären Hydroxygruppen aufweist, und mehrwertige Alkohole entweder Diole der allgemeinen Formel (IIa)

$HO$-$R^6$-$OH$ (IIa)

worin $R^6$ entweder einen unverzweigten Dialkylrest der Formel $-(CH_2)_n-$, wobei n eine ganze Zahl von 3 bis 20, bevorzugt 3 bis 12 ist, bedeutet und beide Hydroxygruppen endständig sind, oder $R^6$ einen unverzweigten, verzweigten oder cyclischen Dialkylrest bedeutet, oder Polyole der allgemeinen Formel (IIb)

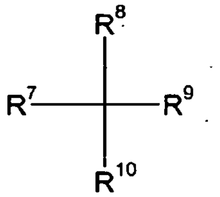

(IIb)

worin die Reste $R^7$, $R^8$, $R^9$, $R^{10}$ unabhängig voneinander Wasserstoff, Hydroxyl, Hydroxymethyl, Hydroxyethyloxymethyl, 1-Hydroxyprop-2-yloxymethyl, 2-Hydroxypropyloxymethyl, Methyl, Ethyl, n-Propyl, Isopropyl, n-Butyl, n-Pentyl, n-Hexyl, 1,2-Dihydroxyethyl, 2-Hydroxyethyl, 3-Hydroxypropyl oder 4-Hydroxybutyl bedeuten und insgesamt 2, 3, oder 4, bevorzugt 2 oder 3, Hydroxygruppen vorhanden sind, und nicht mehr als einer der Reste $R^7$, $R^8$, $R^9$, oder $R^{10}$ gleich Hydroxyl bedeutet, sind,
oder cyclische Carbonate der allgemeinen Formel (III)

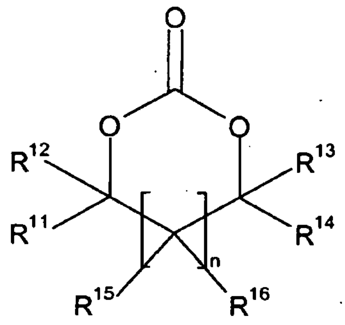

(III)

worin R'', $R^{12}$, $R^{13}$, $R^{14}$, $R^{15}$ und $R^{16}$ unabhängig voneinander Wasserstoff, Methyl, Ethyl, n-Propyl, Isopropyl, n-Butyl, sek.-Butyl oder Isobutyl, und n entweder 0 oder 1 ist,
oder Bisoxazoline der allgemeinen Formel (IV)

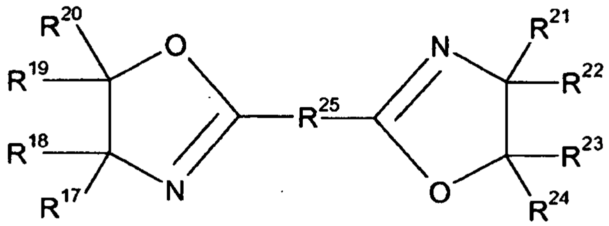

(IV)

worin $R^{17}$, $R^{18}$, $R^{19}$, $R^{20}$, $R^{21}$, $R^{22}$, $R^{23}$ und $R^{24}$ unabhängig voneinander Wasserstoff, Methyl, Ethyl, n-Propyl, Isopropyl, n-Butyl, sek.-Butyl oder Isobutyl, und $R^{25}$ eine Einfachbindung, einen linearen, verzweigten oder cyclischen $C_2$-$C_{12}$-Dialkylrest, oder einen Polyalkoxydiylrest darstellt, welcher aus ein bis zehn Ethylenoxid- und/oder Propylenoxideinheiten aufgebaut ist, wie sie beispielsweise Polyglykoldicarbonsäuren aufweisen.

Die bevorzugten Nachvernetzer sind außerordentlich selektiv. Neben- und Folgereaktionen, die zu flüchtigen und damit übelriechenden Verbindungen führen sind minimiert. Die mit den bevorzugten Nachvernetzern hergestellten wasserabsorbierenden Polymere sind daher auch im angefeuchteten Zustand geruchsneutral.

Dagegen können Epoxyverbindungen bei hohen Temperaturen und Anwesenheit geeigneter Katalysatoren verschiedene Umlagerungsreaktionen eingehen, die beispielsweise zu Aldehyden oder Ketonen führen. Diese können dann weitere Folgereaktionen eingehen, wodurch sich letztendlich übelriechende Verunreinigungen bilden, die wegen ihres Geruchs in Hygieneartikeln unerwünscht sind. Daher sind Epoxyverbindungen oberhalb einer Temperatur von ca. 140 bis 150 °C weniger geeignet für die Nachvernetzung. Bei Amino- bzw. Iminogruppen enthaltenden Nachvernetzern kommt es bei ähnlichen Temperaturen zu noch unübersichtlicheren Umlagerungsreaktionen, wodurch leicht übelriechende Spurenverunreinigungen und bräunliche Produktverfärbungen auftreten.

Mehrwertige Alkohole als Nachvernetzer benötigen aufgrund ihrer geringen Reaktivität hohe Nachvernetzungstemperaturen. Alkohole, die vincinale, geminale, sekundäre und tertiäre Hydroxygruppen aufweisen, bilden dabei im Hygienebereich unerwünschte Nebenprodukte, die zu unangenehmen Gerüchen und/oder Verfärbungen des betreffenden Hygieneartikels während der Herstellung oder des Gebrauchs führen.

Bevorzugte Nachvernetzer der allgemeinen Formel (I) sind 2-Oxazolidone, wie 2-Oxazolidon und N-(2-Hydroxyethyl)-2-oxazolidon, N-Methyl-2-Oxazolidon, N-Acyl-2-oxazolidone, wie N-Acetyl-2-oxazolidon, 2-Oxotetrahydro-1,3-oxazin, bicyclische Amidacetale, wie 5-Methyl-1-aza-4,6-dioxa-bicyclo[3.3.0]octan, 1-Aza-4,6-dioxabicyclo[3.3.0]octan und 5-Isopropyl-1-aza-4,6-dioxa-bicyclo [3.3.0] octan, Bis-2-oxazolidone und Poly-2-oxazolidone.

Besonders bevorzugte Nachvernetzer der allgemeinen Formel (I) sind 2-Oxazolidon, N-Methyl-2-oxazolidon, N-(2-Hydroxyethyl)-2-oxazolidon und N-(2-Hydroxypropyl)-2-oxazolidon.

Bevorzugte Nachvernetzer der allgemeinen Formel (IIa) sind 1,3-Propandiol, 1,5-Pentandiol, 1,6-Hexandiol und 1,7-Heptandiol. Weitere Beispiele für Nachvernetzer der Formel (IIa) sind 1,3-Butandiol, 1,8-Octandiol, 1,9-Nonandiol und 1,10-Decandiol.

Die Diole sind vorzugsweise wasserlöslich, wobei sich die Diole der allgemeinen Formel (IIa) bei 23 °C zu mindestens 30 Gew.-%, bevorzugt zu mindestens 40 Gew.-%, besonders bevorzugt zu mindestens 50 Gew.-%, ganz besonders bevorzugt mindestens zu 60 Gew.-%, in Wasser lösen, wie beispielsweise 1,3-Propandiol und 1,7-Heptandiol. Noch mehr bevorzugt sind solche Nachvernetzer die bei 25°C flüssig sind.

Bevorzugte Nachvernetzer der allgemeinen Formel (IIb) sind Butan-1,2,3-triol, Butan-1,2,4-triol, Glyzerin, Trimethylolpropan, Trimethylolethan, Pentaerythrit, pro Molekül 1-bis 3-fach ethoxyliertes Glyzerin, Trimethylolethan oder

Trimethylolpropan und pro Molekül 1- bis 3-fach propoxyliertes Glyzerin, Trimethylolethan oder Trimethylolpropan. Weiterhin bevorzugt sind 2-fach ethoxyliertes oder propoxyliertes Neopentylglykol. Besonders bevorzugt sind 2-fach und 3-fach ethoxyliertes Glyzerin, Neopentylglykol, 2-Methyl-1,3-propandiol und Trimethylolpropan.

Bevorzugte mehrwertige Alkohole (IIa) und (IIb) weisen bei 23 °C eine Viskosität von weniger als 3000 mPas, vorzugsweise weniger als 1500 mPas, bevorzugt weniger als 1000 mPas, besonders bevorzugt weniger als 500 mPas, ganz besonders bevorzugt weniger als 300 mPas, auf.

Besonders bevorzugte Nachvernetzer der allgemeinen Formel (III) sind Ethylencarbonat und Propylencarbonat.

Ein besonders bevorzugter Nachvernetzer der allgemeinen Formel (IV) ist 2,2' -Bis(2-oxazolin).

Der Nachvernetzer wird typischerweise in einer Menge von höchstens 0,30 Gew.-%, vorzugsweise von höchstens 0,15 Gew.-%, besonders bevorzugt von 0,001 bis 0,095 Gew.-%, jeweils bezogen auf das Grundpolymer, als wässrige Lösung eingesetzt.

Es kann ein einzelner Nachvernetzer aus der obigen Auswahl verwendet werden oder beliebige Gemische verschiedener Nachvernetzer.

Die wässrige Nachvernetzerlösung kann neben dem mindestens einen Nachvernetzer typischerweise noch ein Cosolvens enthalten.

Technisch gut geeignete Cosolventien sind $C_1$-$C_6$-Alkohole, wie Methanol, Ethanol, n-Propanol, Isopropanol, n-Butanol, sec-Butanol, tert.-Butanol oder 2-Methyl-1-propanol, $C_2$-$C_5$-Diole, wie Ethylenglykol, 1,2-Propylenglykol oder 1,4-Butandiol, Ketone, wie Aceton, oder Carbonsäureester, wie Essigsäureethylester. Nachteilig an vielen dieser Cosolventien ist, dass sie typische Eigengerüche aufweisen.

Das Cosolvens selbst ist unter den Reaktionsbedingungen idealerweise kein Nachvernetzer. Es kann jedoch im Grenzfall und abhängig von Verweilzeit und Temperatur dazu kommen, dass das Cosolvens teilweise zur Vernetzung beiträgt. Dies ist insbesondere dann der Fall, wenn der Nachvernetzer relativ träge ist und daher auch selbst sein Cosolvens bilden kann, wie beispielsweise bei Einsatz cyclischer Carbonate der allgemeinen Formel (III), Diole der allgemeinen Formel (IIa) oder Polyole der allgemeinen Formel (IIb). Solche Nachvernetzer können im Gemisch mit reaktiveren Nachvernetzern auch in der Funktion als Cosolvens eingesetzt werden, da die eigentliche Nachvernetzungsreaktion dann bei niedrigeren Temperaturen und/oder kürzeren Verweilzeiten als in Abwesenheit des reaktiveren Vernetzers durchgeführt werden kann. Da das Cosolvens in relativ großen Mengen verwendet wird und auch teilweise im Produkt verbleibt, darf es nicht toxisch sein.

Im erfindungsgemäßen Verfahren eignen sich die Diole der allgemeinen Formel (IIa), die Polyole der allgemeinen Formel (IIb), sowie die cyclischen Carbonate der allgemeinen Formel (III) auch als Cosolventien. Diese Funktion erfüllen sie in Gegenwart eines reaktiven Nachvernetzers der allgemeinen Formel (I) und/oder (IV) und/oder einer Di- oder Triyglycidylverbindung. Bevorzugte Cosolventien im erfindungsgemäßen Verfahren sind jedoch insbesondere die Diole der allgemeinen Formel (IIa), insbesondere, wenn die Hydroxygruppen sterisch durch Nachbargruppen an einer Reaktion behindert werden. Solche Diole eignen sich zwar prinzipiell auch als Nachvernetzer, erfordern dazu jedoch deutlich höhere Reaktionstemperaturen oder gegebenenfalls höhere Einsatzmengen als sterisch ungehinderte Diole.

Weitere im erfindungsgemäßen Verfahren besonders bevorzugte Cosolventien sind die Polyole der allgemeinen Formel (IIb). Insbesondere bevorzugt sind hiervon die 2- bis 3-fach alkoxylierten Polyole. Besonders geeignet als Cosolventien sind aber auch 3- bis 15-fach, ganz besonders 5- bis 10-fach ethoxylierte Polyole auf der Basis von Glyzerin, Trimethylolpropan, Trimethylolethan oder Pentaerythrit. Besonders gut geeignet ist 7-fach ethoxyliertes Trimethylolpropan.

Geeignete sterisch gehinderte und damit reaktionsträge mehrwertige Alkohole sind auch mehrwertige Alkohole mit beliebigen Molekulargewichten, die keine vincinalen, geminalen oder sekundären Hydroxygruppen aufweisen.

Beispiele für solche sterisch gehinderten und daher als Cosolvens besonders bevorzugten Diole der allgemeinen Formel (IIa) sind 2,2-Dimethyl-1,3-propandiol (Neopentylglykol), 2-Methyl-1,3-propandiol und 2,4-Dimethylpentan-2,4-diol.

Weitere geeignete Cosolventien sind Di(trimethylolpropan) sowie 5-Ethyl-1,3-dioxan-5-methanol.

Besonders bevorzugte Kombinationen aus wenig reaktivem Nachvernetzer als Cosolvens und reaktivem Nachvernetzer sind Kombinationen von bevorzugten mehrwertigen Alkoholen, Diolen der allgemeinen Formel (IIa) und Polyolen der allgemeinen Formel (IIb), mit Amidacetalen oder Carbamaten der allgemeinen Formel (I).

Geeignete Kombinationen sind beispielsweise 2-Oxazolidon/1,2-Propandiol und N-(2-Hydroxyethyl)-2-oxazolidon/1,2-Propandiol sowie Ethylenglykoldiglycidylether/1,2-Propandiol.

Ganz besonders bevorzugte Kombinationen sind 2-Oxazolidon/1,3-Propandiol und N-(2-Hydroxyethyl)-2-oxazolidon/1,3-Propandiol.

Weiterhin bevorzugte Kombinationen sind solche mit Ethylenglykoldiglycidylether oder Glyzerindi- oder -triglycidylether mit folgenden Lösemitteln, Cosolventien oder Covernetzern: Isopropanol, 1,3-Propandiol, 2-Methyl-1,3-propandiol, Neopentylglykol, 1,5-Pentandiol, 1,6-Hexandiol, 1,7-Heptandiol oder Gemischen davon.

Das Cosolvens hat vorzugsweise einen Siedepunkt von höchstens 160 °C, besonders bevorzugt von höchstens 140 °C, ganz besonders bevorzugt von höchstens 120 °C, oder vorzugsweise einen Siedepunkt von mindestens 200

°C, besonders bevorzugt von mindestens 220 °C, ganz besonders bevorzugt von mindestens 250 °C.

Im erfindungsgemäßen Verfahren besonders gut geeignete Cosolventien sind daher auch solche, die ein niedrig siedendes Azeotrop mit Wasser oder einem zweiten Cosolvens bilden. Bevorzugt liegt der Siedepunkt dieses Azeotrops bei höchstens 160 °C, besonders bevorzugt höchstens 140 °C, ganz besonders bevorzugt bei höchstens 120 °C. Weiterhin gut geeignet sind wasserdampfflüchtige Cosolventien, da diese ganz oder teilweise mit dem bei der Trocknung verdampfenden Wasser entfernt werden.

Nachvernetzer und Cosolventien mit einem Siedepunkt, der nicht deutlich über oder unter der Temperatur im Nachvernetzungstrockner liegt, führen überraschenderweise oftmals zu wasserabsorbierenden Polymerpartikeln mit einem unerwünschten chemischen Geruch und die Polymere sind stark vergilbt und enthalten oftmals schwarze Stippen und andere Verunreinigungen.

Häufig beträgt die Konzentration des Cosolvens in der wässrigen Nachvernetzerlösung, von 15 bis 50 Gew.-%, vorzugsweise von 15 bis 40 Gew.-%, besonders bevorzugt von 20 bis 35 Gew.-%, bezogen auf die Nachvernetzerlösung. Bei Cosolventien, die mit Wasser nur begrenzt mischbar sind, wird man vorteilhaft die wässrige Nachvernetzerlösung so einstellen, dass nur eine Phase vorliegt, gegebenenfalls durch Erniedrigung der Konzentration des Cosolvens.

In einer bevorzugten Ausführungsform wird kein Cosolvens eingesetzt. Der Nachvernetzer wird dann nur als Lösung in Wasser angewandt, gegebenenfalls unter Zusatz eines Deagglomerationshilfsmittels.

Die Konzentration des mindestens einen Nachvernetzers in der wässrigen Nachvernetzerlösung, beträgt typischerweise 1 bis 20 Gew.-%, vorzugsweise 1,5 bis 10 Gew.-%, besonders bevorzugt 2 bis 5 Gew.-%, bezogen auf die Nachvernetzerlösung.

Die Gesamtmenge der Nachvernetzerlösung bezogen auf Grundpolymer beträgt üblicherweise von 0,3 bis 15 Gew.-%, vorzugsweise von 2 bis 6 Gew.-%.

In einer bevorzugten Ausführungsform wird dem Grundpolymer ein Tensid als Deagglomerationshilfsmittel, beispielsweise Sorbitanmonoester, wie Sorbitanmonococoat und Sorbitanmonolaurat, oder ethoxylierte Varianten davon, wie beispielsweise Polysorbat 20®, zugesetzt. Weitere sehr geeignete Deagglomerationshilfsmittel stellen die ethoxylierten und alkoxylierten Derivate des 2-Propylheptanols dar, die unter den Handelsmarken Lutensol XL® und Lutensol XP® vertrieben werden (BASF Aktiengesellschaft, DE).

Alle anionischen, kationischen, nichtionischen und amphoteren Tenside sind als Deagglomerationshilfsmittel geeignet, bevorzugt sind jedoch aus Hautverträglichkeitsgründen nicht-ionische und amphotere Tenside. Das Tensid kann auch Stickstoff enthalten.

Das Deagglomerationshilfsmittel kann getrennt dosiert oder der Nachvernetzerlösung zugesetzt werden. Vorzugsweise wird das Deagglomerationshilfsmittel der Nachvernetzerlösung einfach zugesetzt.

Die Einsatzmenge des Deagglomerationshilfsmittels bezogen auf Grundpolymer beträgt beispielsweise 0 bis 0,1 Gew.-%, vorzugsweise 0 bis 0,01 Gew.-%, besonders bevorzugt 0 bis 0,002 Gew.-%. Vorzugsweise wird das Deagglomerationshilfsmittel so dosiert, dass die Oberflächenspannung eines wässrigen Extrakts des gequollenen Grundpolymers und/oder des gequollenen nachvernetzten wasserabsorbierenden Polymeren bei 23 °C mindestens 0,060 N/m, vorzugsweise mindestens 0,062 N/m, besonders bevorzugt mindestens 0,065 N/m, und vorteilhaft höchstens 0,072 N/m beträgt.

Das im erfindungsgemäßen Verfahren eingesetzte getrocknete Grundpolymer weist typischerweise einen Restfeuchtegehalt nach der Trocknung und vor Aufbringen der Nachvernetzungslösung von 0 bis 13 Gew.-%, bevorzugt 2 bis 9 Gew.-% auf. Optional kann dieser Feuchtegehalt aber auch beispielsweise durch Aufbringen von Wasser in einem vorgeschalteten Sprühmischer auf bis zu 75 Gew.-% erhöht werden. Der Feuchtegehalt wird gemäß der von der EDANA (European Disposables and Nonwovens Association) empfohlenen Testmethode Nr. WSP 230.2-05 "Moisture content" bestimmt. Eine solche Erhöhung des Feuchtegehalts führt zu einer leichten Vorquellung des Grundpolymers und verbessert die Verteilung des Vernetzers auf der Oberfläche sowie die Durchdringung der Polymerpartikel.

Die im erfindungsgemäßen Verfahren einsetzbaren Sprühdüsen unterliegen keiner Beschränkung. Geeignete Düsen und Zerstäubungssysteme sind beispielsweise in den folgenden Literaturstellen beschrieben: Zerstäuben von Flüssigkeiten, Expert-Verlag, Bd. 660, Reihe Kontakt & Studium, Thomas Richter (2004) sowie in Zerstäubungstechnik, Springer-Verlag, VDI-Reihe, Günter Wozniak (2002). Einsetzbar sind mono- und polydisperse Sprühsysteme. Unter den polydispersen Systemen sind Einstoff-Druckdüsen (strahl- oder lamellenbildend), Rotationszerstäuber, Zweistoffzerstäuber, Ultraschallzerstäuber und Pralldüsen geeignet. Bei den Zweistoffzerstäubern kann die Mischung der Flüssigkeits- mit der Gasphase sowohl innenliegend als auch außenliegend erfolgen. Das Sprühbild der Düsen ist unkritisch und kann jede beliebige Form annehmen, beispielsweise Rundstrahl-, Flachstrahl-, Weitwinkel-Rundstrahl- oder Kreisring-Spritzbild. Vorteilhaft ist die Verwendung eines nicht-oxidierenden Gases falls Zweistoffzerstäuber eingesetzt werden, besonders bevorzugt sind Stickstoff, Argon oder Kohlendioxid. Derartigen Düsen kann die zu versprühende Flüssigkeit unter Druck zugeführt werden. Die Zerteilung der zu versprühenden Flüssigkeit kann dabei dadurch erfolgen, dass sie nach Erreichen einer bestimmten Mindestgeschwindigkeit in der Düsenbohrung entspannt wird. Ferner können für den erfindungsgemäßen Zweck auch Einstoffdüsen, wie beispielsweise Schlitzdüsen oder Drallkammern (Vollkegeldüsen) verwendet werden (beispielsweise von Düsen-Schlick GmbH, DE, oder von Spraying Systems Deutschland

GmbH, DE). Derartige Düsen sind auch in der EP 0 534 228 A1 und der EP 1 191 051 A2 beschrieben.

Im Anschluß an das Aufsprühen werden die Polymerpartikel thermisch nachvernetzt.

Das Aufsprühen der Nachvernetzerlösung wird vorzugsweise in Mischern mit bewegten Mischwerkzeugen, wie Schneckenmischer, Paddelmischer, Scheibenmischer, Pflugscharmischer und Schaufelmischer, durchgeführt. Besonders bevorzugt sind Vertikalmischer, ganz besonders bevorzugt sind Pflugscharmischer und Schaufelmischer. Geeignete Mischer sind beispielsweise Lödige-Mischer, Bepex-Mischer, Nauta-Mischer, Processall-Mischer und Schugi-Mischer.

Die thermische Nachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex-Trockner und Nara-Trockner. Überdies können auch Wirbelschichttrockner, beispielsweise Carman-Trockner, eingesetzt werden.

Die thermische Nachvernetzung kann im Mischer selbst erfolgen, beispielsweise durch Beheizung des Mantels oder Einblasen warmer Inertgase. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Besonders bevorzugt wird die Nachvernetzerlösung in einem Hochgeschwindigkeitsmischer, beispielsweise vom Typ Schugi-Flexomix oder Turbolizer, auf das Grundpolymer aufgebracht und in einem Reaktionstrockner, beispielsweise vom Typ Nara-Paddle-Dryer oder einem Scheibentrockner, thermisch nachvernetzt. Das eingesetzte Grundpolymer kann aus vorhergehenden Prozeßschritten noch eine Temperatur von 10 bis 120°C aufweisen, die Nachvernetzerlösung kann eine Temperatur von 0 bis 150°C aufweisen. Insbesondere kann die Nachvernetzerlösung zur Verminderung der Viskosität erwärmt werden. Bevorzugt zur Nachvernetzung und Trocknung ist dabei der Temperaturbereich von 30 bis 220°C, insbesondere 120 bis 210 °C, besonders bevorzugt 145 bis 190°C. Die bevorzugte Verweilzeit bei dieser Temperatur im Reaktionsmischer oder Trockner beträgt unter 100 Minuten, besonders bevorzugt unter 70 Minuten, am meisten bevorzugt unter 40 Minuten. Falls ein Wirbelschichttrockner eingesetzt wird beträgt die Verweilzeit vorzugsweise unter 30 Minuten, besonders bevorzugt unter 20 Minuten, ganz besonders bevorzugt unter 10 Minuten.

Der Nachvernetzungstrockner wird im erfindungsgemäßen Verfahren vorzugsweise während der thermischen Nachvernetzung mit einem Inertgas gespült, um die Dämpfe zu entfernen und oxidierende Gase, wie Luftsauerstoff, zu verdrängen. Zur Unterstützung der Trocknung werden der Trockner und die angeschlossenen Aggregate thermisch gut isoliert und möglichst vollständig beheizt.

Geeignete Inertgase sind beispielsweise Stickstoff, Kohlendioxid, Argon, Wasserdampf, wobei Stickstoff bevorzugt ist. Die Inertgasmenge beträgt vorzugsweise 0,0001 bis 10 $m^3$, bevorzugt 0,001 bis 5$m^3$, besonders bevorzugt 0,005 bis 1 $m^3$, und ganz besonders bevorzugt 0,005 bis 0,1 $m^3$, bezogen auf 1 kg wasserabsorbierende Polymerpartikel.

Inertgase im erfindungsgemäßen Verfahren sind bei der Nachvernetzungstemperatur und gegebenem Druck im Nachvernetzungstrockner gasförmig vorliegende Stoffe, die unter diesen Bedingungen nicht oxidierend auf die Bestandteile der trocknenden Polymerpartikel wirken.

Zur Erzeugung möglichst weißer Polymerpartikel wird im erfindungsgemäßen Verfahren der Gasraum im Trockner möglichst frei von oxidierenden Gasen gehalten.

Es ist erfindungsgemäß möglich die Polymerpartikel über Kontaktflächen im Trockner zu beheizen, oder über zugeführtes warmes Inertgas, oder über eine Mischung eines oder mehrerer Inertgase mit Wasserdampf, oder nur mit Wasserdampf allein. Bei Zufuhr der Wärme über Kontaktflächen ist es möglich die Reaktion bei leichtem oder vollständigem Unterdruck unter Inertgas durchzuführen. Bei Verwendung von Wasserdampf zum direkten Beheizen der Polymerpartikel ist es erfindungsgemäß wünschenswert den Trockner bei Normaldruck oder Überdruck zu betreiben. In diesem Fall kann es sinnvoll sein den Nachvernetzungsschritt in einen Aufheizschritt und einen Reaktionsschritt unter Inertgas aber ohne Wasserdampf aufzuspalten. Dies kann in einem oder mehreren Apparaten realisiert werden. Erfindungsgemäß können die Polymerpartikel schon im Nachvernetzungsmischer mit Wasserdampf aufgeheizt werden.

Im erfindungsgemäßen Verfahren kann das Inertgas, wenn es nicht Wasserdampf enthält, über Düsen in den Nachvernetzungstrockner eingeblasen werden, besonders bevorzugt wird das Inertgas aber bereits im oder kurz vor dem Nachvernetzungsmischer über Düsen dem Polymerpartikelstrom zugegeben. Beispielsweise kann der Sauerstoffgehalt in der Atmosphäre des Nachvernetzungstrockners oder des Nachvernetzungsmischers mittels Sauerstoffsonden kontrolliert werden.

Selbstverständlich können mit den Dämpfen abgeführte Cosolventien außerhalb des Nachvernetzungstrockners wieder kondensiert und gegebenenfalls recyclisiert werden.

Nach Abschluß der thermischen Nachvernetzung werden die getrockneten wasserabsorbierenden Polymerpartikel abgekühlt. Vorzugsweise überführt man dazu die warmen und trockenen Polymerpartikel im kontinuierlichen Betrieb in einen nachgeschalteten Kühler. Dieser kann beispielsweise ein Scheibenkühler, ein Nara-Paddle-Kühler oder ein Schneckenkühler sein. Gekühlt wird dabei über die Wände und gegebenenfalls die Rührorgane des Kühlers, welche von einem geeigneten Kühlmedium wie beispielsweise Warm- oder Kaltwasser durchströmt werden. Wahlweise kann jedoch auch ein Wirbelschichtkühler eingesetzt werden. Zweckmäßig können im Kühler Wasser oder wässrige Lösungen

von Additiven, beispielsweise die erfindungsgemäßen Metallsalze als wässrige Lösung, aufgesprüht werden; wodurch sich die Effizienz der Kühlung erhöht (partielle Wasserverdampfung) und der Restfeuchtegehalt im Fertigprodukt kann auf vorzugsweise 0 bis 6 Gew.-%, besonders bevorzugt 0,01 bis 4 Gew.-%, ganz besonders bevorzugt 0,1 bis 3 Gew.-%, eingestellt werden. Der erhöhte Restfeuchtegehalt verringert den Staubgehalt des Produkts.

Optional kann jedoch im Kühler auch nur gekühlt werden und die Zugabe von Wasser und Additiven in einem nachgeschalteten separaten Mischer durchgeführt werden. Die Kühlung stoppt die Reaktion durch Unterschreiten der Reaktionstemperatur und die Temperatur braucht insgesamt nur so weit abgesenkt werden, dass das Produkt sich problemlos in Plastiksäcke oder in Silo-LKW abpacken läßt.

Optional können auf die Oberfläche der wasserabsorbierenden Polymerpartikel im Herstellverfahren in jedem Prozeßschritt bei Bedarf alle bekannten Beschichtungen, wie filmbildende Polymere, thermoplastische Polymere, Dendrimere, polykationische Polymere (wie beispielsweise Polyvinylamin, Polyethylenimin oder Polyallylamin), wasserunlösliche polyvalente Metallsalze, wie beispielsweise Magnesiumcarbonat, Magnesiumoxid, Magnesiumhydroxid, Calciumcarbonat, Calciumsulfat oder Calciumphosphat, alle dem Fachmann bekannten wasserlöslichen mono- oder polyvalenten Metallsalze, wie beispielsweise Aluminiumsulfat, Natrium-, Kalium-, Zirconium- oder Eisensalze, oder hydrophile anorganische Partikel, wie Tonminerale, pyrogene Kieselsäure, Aluminiumoxid und Magnesiumoxid, zusätzlich aufgebracht werden. Dadurch können zusätzliche Effekte, beispielsweise eine verringerte Verbackungsneigung, verbesserte Verarbeitungseigenschaften oder eine weiter gesteigerte Flüssigkeitsleitfähigkeit (SFC) erreicht werden. Wenn die Additive in Form von Dispersionen eingesetzt und aufgesprüht werden, dann werden sie bevorzugt als wässrige Dispersionen eingesetzt, und es wird bevorzugt noch zusätzlich ein Entstaubungsmittel zur Fixierung des Additivs auf der Oberfläche des wasserabsorbierenden Polymers aufgebracht. Das Entstaubungsmittel wird dann entweder direkt der Dispersion des anorganischen pulvrigen Additivs hinzugefügt, optional kann es auch als separate Lösung vor, während, oder nach dem Auftrag des anorganischen pulvrigen Additivs durch Aufsprühen hinzugefügt werden. Am meisten bevorzugt ist die gleichzeitige Aufsprühung von Nachvernetzungsmittel, Entstaubungsmittel und pulvrigem anorganischen Additiv in der Nachvernetzung. In einer weiteren bevorzugten Verfahrensvariante wird das Entstaubungsmittel aber separat im Kühler zugegeben, beispielsweise durch Aufsprühen von oben, unten oder von der Seite. Besonders geeignete Entstaubungsmittel, die auch zur Fixierung pulvriger anorganischer Additive an der Oberfläche der wasserabsorbierenden Polymerpartikel dienen können, sind Polyethylenglykole mit einem Molekulargewicht von 400 bis 20000 g/mol, Polyglyzerin, 3- bis 100-fach ethoxylierte Polyole, wie Trimethylolpropan, Glyzerin, Sorbitol und Neopentylglykol. Besonders geeignet sind 7- bis 20-fach ethoxyliertes Glyzerin oder Trimethylolpropan, wie beispielsweise Polyol TP 70® (Perstorp, SE). Letztere haben insbesondere den Vorteil, dass sie die Oberflächenspannung eines wässrigen Extrakts der wasserabsorbierenden Polymerpartikel nur unwesentlich herabsetzen.

In einer besonders bevorzugten Ausführungsform der Erfindung werden Nachvernetzer (vorzugsweise 2-Oxazolidon oder N-(2-Hydroxyethyl)-2-oxazolidon und/oder 1,3-Propandiol), organisches Lösemittel (vorzugsweise Isopropanol), Aluminiumlaktat, und gegebenenfalls noch etwas Tensid (bevorzugt Span® 20) mit Wasser gelöst und dann mittels eines Sprühmischers (vorzugsweise Schugi-Mix) auf die Polymerpartikel mittels einer Zweistoffdüse oder Einstoffdüse aufgebracht, wobei sowohl der Sprühmischer als auch die nachgeschaltete thermische Nachvernetzung so mit Inertgas (vorzugsweise Stickstoff) gespült werden, dass der Volumenanteil Sauerstoff in diesen Aggregaten weniger als 14 Vol.-%, vorzugsweise weniger als 10 Vol.-%, besonders bevorzugt weniger als 5 Vol.-%, ganz besonders bevorzugt weniger als 1 Vol.-%, beträgt.

In einer weiteren besonders bevorzugten Ausführungsform der Erfindung werden Nachvernetzer (vorzugsweise 2-Oxazolidon oder N-(2-Hydroxyethyl)-2-oxazolidon und/oder 1,3-Propandiol), organisches Lösemittel (vorzugsweise Isopropanol), Aluminiumlaktat und gegebenenfalls noch etwas Tensid (bevorzugt Span® 20) mit Wasser gelöst und dann mittels eines Sprühmischers (vorzugsweise Schugi-Mix) auf die Polymerpartikel mittels einer Zweistoffdüse oder Einstoffdüse aufgebracht, wobei sowohl der Sprühmischer als auch der nachgeschaltete Nachvernetzungstrockner so mit Inertgas (vorzugsweise Stickstoff) gespült werden, dass der Volumenanteil Sauerstoff in diesen Aggregaten weniger als 14 Vol.-%, vorzugsweise weniger als 10 Vol.-%, besonders bevorzugt weniger als 5 Vol.-%, ganz besonders bevorzugt weniger als 1 Vol.-%, beträgt. Über eine separate Zuführung wird zeitgleich zu dieser Nachvernetzungslösung eine gesättigte wässerige Lösung von Calciumhydroxid mit darin fein dispergiertem festen Calciumhydroxid aufgesprüht.

In noch einer weiteren besonders bevorzugten Ausführungsform der Erfindung werden Nachvernetzer (vorzugsweise Ethylengykoldigylcidylether oder 2-Oxazolidon oder N-(2-Hydroxyethyl)-2-oxazolidon und/oder 1,3-Propandiol und /oder 1,2-Propandiol), gegebenenfalls organisches Lösemittel (vorzugsweise Isopropanol), ein wasserlösliches Calciumsalz (bevorzugt Calciumlaktat) und Aluminiumlaktat und gegebenenfalls noch etwas Tensid (bevorzugt Span® 20) mit Wasser gelöst und dann mittels eines Sprühmischers (vorzugsweise Schugi-Mix) auf die Polymerpartikel mittels einer Zweistoffdüse oder Einstoffdüse aufgebracht, wobei sowohl der Sprühmischer als auch der nachgeschaltete Nachvernetzungstrockner so mit Inertgas (vorzugsweise Stickstoff) gespült werden, dass der Volumenanteil Sauerstoff in diesen Aggregaten weniger als 14 Vol.-%, vorzugsweise weniger als 10 Vol.-%, besonders bevorzugt weniger als 5 Vol.-%, ganz besonders bevorzugt weniger als 1 Vol.-%, beträgt.

Ein weitere Gegenstand der vorliegenden Erfindung sind die gemäß dem erfindungsgemäßen Verfahren erhältlichen wasserabsorbierenden Polymerpartikel.

Die erfindungsgemäßen wasserabsorbierenden Polymerpartikel weisen typischerweise eine Zentrifugenretentionskapazität (CRC) von mindestens 25 g/g, vorzugsweise von mindestens 26 g/g, bevorzugt mindestens 27 g/g, besonders bevorzugt mindestens 28 g/g, ganz besonders bevorzugt mindestens 29 g/g, und üblicherweise nicht über 40 g/g, auf. Die Zentrifugenretentionskapazität (CRC) wird gemäß der von der EDANA (European Disposables and Nonwovens Association) empfohlenen Testmethode Nr. WSP 241.2-05 "Centrifuge retention capacity" bestimmt.

Die erfindungsgemäßen wasserabsorbierenden Polymerpartikel weisen typischerweise eine Absorption unter Druck von 4,83 kPa (AUL0.7psi) von mindestens 21 g/g, vorzugsweise mindestens 22 g/g, bevorzugt mindestens 23 g/g, besonders bevorzugt mindestens 24 g/g, ganz besonders bevorzugt mindestens 25 g/g, und üblicherweise nicht über 30 g/g, auf. Die Absorption unter Druck (AUL0.7psi) wird analog der von der EDANA (European Disposables and Nonwovens Association) empfohlenen Testmethode Nr. WSP 242.2-05 "Absorption under pressure" bestimmt.

Die erfindungsgemäßen wasserabsorbierenden Polymerpartikel weisen typischerweise eine Flüssigkeitsweiterleitung (SFC) von mindestens $30x10^{-7}cm^3s/g$, vorzugsweise mindestens $45x10^{-7}cm^3s/g$, bevorzugt mindestens $80x10^{-7}cm^3s/g$, besonders bevorzugt mindestens $100x10^{-7}cm^3s/g$, ganz besonders bevorzugt mindestens $140x10^{-7}cm^3s/g$, und üblicherweise nicht über $700x10^{-7}cm^3s/g$, auf.

Nach dem erfindungsgemäßen Verfahren werden wasserabsorbierende Polymerpartikel mit einem hohen Weißegrad erhalten. Die erhaltenen wasserabsorbierenden Polymerpartikel können bei erhöhter Umgebungstemperatur und hoher Luftfeuchte gelagert werden und verfärben sich im Vergleich zu normalen wasserabsorbierenden Polymerpartikeln nur sehr langsam und sind daher als Polymerpartikel und nach Verarbeitung als Hygieneartikel besser lager- und länger verwendungsfähig. Insbesondere weisen wasserabsorbierende Polymerpartikel, die mit beiden Salzen (Calcium- und Aluminiumsalz) beschichtet werden, eine hohe Flüssigkeitsleitfähigkeit (SFC) bei hoher Absorptionsfähigkeit (CRC und AUL0.7psi) sowie guter Farbstabilität und hohem Weißegrad auf.

Der L-Wert der weißen Polymerpartikel beträgt im nicht gelagerten Zustand typischerweise mindestens 75, vorzugsweise mindestens 80, besonders bevorzugt mindestens 85, ganz besonders bevorzugt mindestens 90, und höchstens 100.

Der a-Wert der weißen Polymerpartikel beträgt im nicht gelagerten Zustand typischerweise von -2,5 bis +2,5, vorzugsweise von -2,0 bis +2,0, besonders bevorzugt von -1,5 bis +1,5, ganz besonders bevorzugt von -0,5 bis +0,5.

Der b-Wert der weißen Polymerpartikel beträgt im nicht gelagerten Zustand typischerweise von 0 bis 10, vorzugsweise von 2 bis 8, besonders bevorzugt von 3 bis 7, ganz besonders bevorzugt von 4 bis 6,5.

Nach "Lagertest" bei erhöhter Temperatur und hoher Luftfeuchte weisen die erfindungsgemäßen wasserabsorbierenden Polymerpartikel nach Messung für die L- und a- Werte Ergebnisse im Bereich der Proben im nicht-gelagerten Zustand auf, und weisen nach 100 Stunden Lagerung noch b-Werte von vorzugsweise nicht mehr als 12, besonders bevorzugt nicht mehr als 10, auf sowie nach 300 Stunden Lagerung noch b-Werte von vorzugsweise nicht mehr als 15, besonders bevorzugt nicht mehr als 12, auf. Ein b-Wert oberhalb 12 ist in Damenhygieneartikeln und ultradünnen Windeln kritisch; ein b-Wert von mehr als 15 ist auch bereits in üblichen Windeln kritisch da diese Verfärbung vom Verbraucher bei Benutzung wahrgenommen werden kann.

Weiterhin sind die erfindungsgemäßen wasserabsorbierenden Polymerpartikel weitehend frei von Verbindungen, die insbesondere während des Gebrauchs, zu unangenehmen Gerüchen führen.

Ein weiterer Gegenstand der vorliegenden Erfindung sind Hygieneartikel, enthaltend erfindungsgemäße wasserabsorbierende Polymerpartikel, vorzugsweise ultradünne Windeln, enthaltend eine absorbierende Schicht bestehend aus 50 bis 100 Gew.-%, vorzugsweise 60 bis 100 Gew.-%, bevorzugt 70 bis 100 Gew.-%, besonders bevorzugt 80 bis 100 Gew.-%, ganz besonders bevorzugt 90 bis 100 Gew.-%, erfindungsgemäßer wasserabsorbierender Polymerpartikel, wobei die Umhüllung der absorbierenden Schicht selbstverständlich nicht berücksichtigt ist.

Ganz besonders vorteilhaft sind die erfindungsgemäßen wasserabsorbierenden Polymerpartikel auch zur Herstellung von Laminaten und Kompositstrukturen, wie sie beispielsweise in der US 2003/0181115 sowie der US 2004/0019342 beschrieben sind, geeignet. Zusätzlich zu den in beiden Schriften zur Herstellung solcher neuer absorbierenden Strukturen beschriebenen Schmelzklebern und insbesondere den in der US 2003/0181115 beschriebenen Fasern aus Schmelzklebern, an die die wasserabsorbierenden Polymerpartikel gebunden ist, eignen sich die erfindungsgemäßen wasserabsorbierenden Polymerpartikel auch zur Herstellung von vollkommen analogen Strukturen unter Verwendung von UV-vernetzbaren Schmelzklebern, welche beispielsweise als AC-Resin® (BASF Aktiengesellschaft, DE) vertrieben werden. Diese UV-vernetzbaren Schmelzkleber haben den Vorteil bereits bei 120 bis 140 °C verarbeitbar zu sein, daher sind sie mit vielen thermoplastischen Substraten besser kompatibel. Ein weiterer wesentlicher Vorteil besteht darin, dass UV-vernetzbare Schmelzkleber toxikologisch sehr unbedenklich sind und auch keine Ausdünstungen in den Hygieneartikeln verursachen. Ein ganz wesentlicher Vorteil, im Zusammenhang mit den erfindungsgemäßen wasserabsorbierenden Polymerpartikeln, ist die Eigenschaft der UV-vernetzbaren Schmelzkleber während der Verarbeitung und Vernetzung nicht zur Vergilbung zu neigen. Dies ist insbesondere von Vorteil, wenn ultradünne oder teilweise transparente Hygieneartikel hergestellt werden sollen. Die Kombination der erfindungsgemäßen wasserabsorbierenden Polymerpartikel mit UV-vernetzbaren Schmelzklebern ist daher besonders vorteilhaft. Geeignete UV-vernetzbare

Schmelzkleber sind beispielsweise beschrieben in EP 0 377 199 A2, EP 0 445 641 A1, US 5,026,806, EP 0 655 465 A1 und EP 0 377 191 A2.

Zur Bestimmung der Güte der Nachvernetzung werden die getrockneten wasserabsorbierenden Polymerpartikel mit den nachfolgend beschrieben Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

Zentrifugenretentionskapazität (CRC Centrifuge Retention Capacity)

Die Zentrifugenretentionskapazität wird gemäß der von der EDANA (European Disposables and Nonwovens Association) empfohlenen Testmethode Nr. WSP 241.2-05 "Centrifuge retention capacity" bestimmt.

Absorption unter Druck (AUL0.3psi Absorbency Under Load)

Die Absorption unter einem Druck von 2,07 kPa (0,3 psi) der wasserabsorbierenden Polymerpartikel wird gemäß der von der EDANA (European Disposables and Nonwovens Association) empfohlenen Testmethode Nr. WSP 242.2-05 "Absorption under pressure" bestimmt.

Absorption unter Druck (AUL0.7psi Absorbency Under Load)

Die Absorption unter einem Druck von 4,83 kPa (0,7 psi) der wasserabsorbierenden Polymerpartikel wird analog der von der EDANA (European Disposables and Nonwovens Association) empfohlenen Testmethode Nr. WSP 242.2-05 "Absorption under pressure" bestimmt, wobei ein Gewicht mit 49 g/cm$^2$ (0,7 psi) statt eines Gewichts mit 21 g/cm$^2$ (0,3 psi) verwendet wird.

Flüssigkeitsweiterleitung (SFC Saline Flow Conductivity)

Die Flüssigkeitsweiterleitung einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluß wird automatisch erfaßt.

Die Flüssigkeitsweiterleitung (SFC) wird wie folgt berechnet:

$$\text{SFC [cm}^3\text{s/g]} = (\text{Fg(t=0)xL0})/(\text{dxAxWP}),$$

wobei Fg(t=0) der Durchfluß an NaCl-Lösung in g/s ist, der anhand einer linearen Regressionsanalyse der Daten Fg(t) der Durchflußbestimmungen durch Extrapolation gegen t=0 erhalten wird, L0 die Dicke der Gelschicht in cm, d die Dichte der NaCl-Lösung in g/cm$^3$, A die Fläche der Gelschicht in cm$^2$ und WP der hydrostatische Druck über der Gelschicht in dyn/cm$^2$ darstellt.

Extrahierbare 16h

Der Gehalt an extrahierbaren Bestandteilen der wasserabsorbierenden Polymerpartikel wird gemäß der von der EDANA (European Disposables and Nonwovens Association) empfohlenen Testmethode Nr. WSP 270.2-05 "Extractables" bestimmt.

Feuchtegehalt des Hydrogels

Der Wassergehalt der wasserabsorbierenden Polymerpartikel wird gemäß der von der EDANA (European

Disposables and Nonwovens Association) empfohlenen Testmethode Nr. WSP 230.2-05 "Moisture content" bestimmt.

CIE-Farbzahl (L a b)

Die Farbmessung wurde entsprechend dem CIELAB-Verfahren (Hunterlab, Band 8, Jahrgang 1996, Heft 7, Seiten 1 bis 4) durchgeführt. Dabei werden die Farben über die Koordinaten L, a und b eines dreidimensionalen Systems beschrieben. Dabei gibt L die Helligkeit an, wobei L = 0 schwarz und L = 100 weiß bedeutet. Die Werte für a und b geben die Position der Farbe auf den Farbachsen rot/grün bzw. gelb/blau an, wobei +a für rot, -a für grün, +b für gelb und -b für blau steht.

Die Farbmessung entspricht dem Dreibereichsverfahren nach DIN 5033-6.

Verwendet wurde ein Colorimeter: "Modell LabScan XE S/N LX17309" (HunterLab, Reston, US).

Lagertest

Messung 1: Eine Glasschale mit 9 cm Innendurchmesser und 1,5 cm Höhe wird mit wasserabsorbierenden Polymerpartikeln überfüllt und dann mit einem Messer über den Rand glatt gestrichen und die CIE-Farbzahlen bestimmt.

Messung 2: Eine Glasschale mit 9 cm Innendurchmesser und 1,5 cm Höhe wird mit wasserabsorbierenden Polymerpartikeln überfüllt und dann mit einem Messer über den Rand glatt gestrichen. Die Schale wird mit einem passenden Glasdeckel abgedeckt und die CIE-Farbzahlen bestimmt.

Messung 3 & Lagerung: Eine Glasschale mit 9 cm Innendurchmesser und 1,5 cm Höhe wird mit 30 g wasserabsorbierenden Polymerpartikeln gefüllt und dann glatt gestrichen. Die Schale wird dann offen in einen temperierten Klimaschrank bei konstanter Luftfeuchte gestellt. Die Partikel quellen dabei etwas an und unterliegen für einen vorbestimmten Zeitraum diesen klimatischen Bedingungen. Es wird bei 60 °C und 90 % relativer Luftfeuchte gelagert.

Die Schale wird nach Ablauf der Lagerzeit herausgenommen, mit einem passenden Glasdeckel abgedeckt und die CIE-Farbzahlen bestimmt.

Die EDANA-Testmethoden sind beispielsweise erhältlich bei der European Disposables and Nonwovens Association, Avenue Eugene Plasky 157, B-1030 Brüssel, Belgien.

Beispiele

Beispiel 1 (Vergleichsbeispiel)

In einen Lödige-Pflugscharkneter Typ VT 5R-MK (5 l Volumen) wurden 459 g Wasser, 213,9 g Acrylsäure (stabilisiert mit 0,02 Gew.-% Hydrochinonmonomethylether), 1924,9 g einer 37,3 gew.-%igen Natriumacrylatlösung (100 mol-% neutralisiert), die zuvor zwecks Entfernung von Hydrochinonmonomethylether über Aktivkohle filtriert wurde, sowie 2,52 g 3-fach ethxoyliertem Glycerintriacrylat vorgelegt und unter Durchperlen von Stickstoff 20 Minuten inertisiert. Der Gehalt an Hydrochinomonomethylether, bezogen auf Acrylsäure, betrug ca. 0,005 Gew.-%. Die Reaktionsmischung wurde dabei von außen so gekühlt, dass die nachfolgende Initiatorzugabe bei ca. 20 °C erfolgte. Schließlich wurden in den Kneter unter Rühren noch 2,139 g Natriumpersulfat (gelöst in 12,12 g Wasser), 0,046 g Ascorbinsäure (gelöst in 9,12 g Wasser) und 0,127 g 30gew.-%iges Wasserstoffperoxid (gelöst in 1,15 g Wasser) rasch hintereinander hinzugefügt. Die Reaktion setzte zügig ein und bei Erreichen einer Innentemperatur von 30°C wurde der Mantel mit 80 °C heißem Wärmeträgermedium beheizt, um die Reaktion möglichst adiabat zu Ende zu führen. Nach Erreichen der Maximaltemperatur wurde dann mittels Kühlflüssigkeit (-12°C) das entstandene Gel auf unter 50°C herabgekühlt und dann ausgetragen.

Das Gel wurde auf zwei Bleche mit Drahtboden verteilt und bei 140 °C und 250 mbar im Vakuumtrockenschrank getrocknet. Anschließend wurde mit einer Ultrazentrifugalmühle zerkleinert und das Produkt auf eine Korngröße von 150 bis 850 μm abgesiebt.

Das so hergestellte Grundpolymer hatte folgende Eigenschaften:

CRC = 36,5 g/g
AUL0.3psi = 8,3 g/g
Extrahierbare 16 h = 12,8 Gew.-%
Restfeuchte = 2,6 Gew.-%

| Farbzahl | Messung 1 | Messung 2 | Messung 3 (nach 100 h) | Messung 3(nach 300 h) |
|---|---|---|---|---|
| L | 91,2 | 86,2 | 76,0 | 70,0 |

(fortgesetzt)

| Farbzahl | Messung 1 | Messung 2 | Messung 3 (nach 100 h) | Messung 3 (nach 300 h) |
|---|---|---|---|---|
| a | -1,3 | -1,8 | 0,8 | 2,5 |
| b | 6,9 | 7,2 | 11,5 | 16,0 |

Beispiel 2 (Vergleichsbeispiel)

Das in Beispiel 1 hergestellte Grundpolymer (20 g) wurde im Waring-Labormischer auf mittlerer Rührstufe mittels einer Injektionsspritze mit einer Nachvernetzerlösung, bestehend aus 0,02 g N-(2-Hydroxyethyl)-2-oxazolidinon, 0,60 g Wasser und 0,30 g 1,3-Propandiol, besprüht. Die feuchten Polymerpartikel wurden mit einem Spatel nochmals homogenisiert und anschließend in einer Petrischale mit einem Innendurchmesser von 18,5 cm gleichmäßig verteilt und in einem Vakuumtrockenschrank (Heraeus VACUTHERM VT 6060M; Kendro Laboratory Products GmbH, DE) für 60 Minuten bei 175 °C wärmebehandelt. Dabei wurde mit ca. 1200 1/h Stickstoff bei ca. 1013 mbar überlagert, wodurch ein Sauerstoffpartialdruck von <10 mbar erzeugt wurde.

Die nachvernetzten Polymerpartikel wurden über ein 850 μm Sieb von Klumpen befreit und analysiert.

Die so hergestellten nachvernetzten Polymerpartikel hatten folgende Eigenschaften:

CRC = 31,0 g/g
AUL0.7psi = 24,5 g/g
SFC = $30x10^{-7}cm^3s/g$

| Farbzahl | Messung 1 | Messung 2 | Messung 3 (nach 100 h) | Messung 3 (nach 300 h) |
|---|---|---|---|---|
| L | 89,2 | 84,3 | 73,9 | 65,0 |
| a | -1,0 | -1,5 | 2,1 | 4,5 |
| b | 7,9 | 8,2 | 13,5 | 20,5 |

Beispiel 3 (Vergleichsbeispiel)

Das in Beispiel 1 hergestellte Grundpolymer (20 g) wurde im Waring-Labormischer auf mittlerer Rührstufe mittels einer Injektionsspritze mit einer Nachvernetzerlösung, bestehend aus 0,02 g N-(2-Hydroxyethyl)-2-oxazolidinon, 0,60 g Wasser und 0,30 g 1,3-Propandiol und 0,20 g Calciumhydroxid dispergiert in dieser Lösung, besprüht. Die feuchten Polymerpartikel wurden mit einem Spatel nochmals homogenisiert und anschließend in einer Petrischale mit einem Innendurchmesser von 18,5 cm gleichmäßig verteilt und in einem Vakuumtrockenschrank (Heraeus VACUTHERM VT 6060M; Kendro Laboratory Products GmbH, DE) für 60 Minuten bei 175 °C wärmebehandelt. Dabei wurde mit ca. 1200 1/h Stickstoff bei ca. 1013 mbar überlagert, wodurch ein SauerStoffpartialdruck von <10 mbar erzeugt wurde.

Die nachvernetzten Polymerpartikel wurden über ein 850 μm Sieb von Klumpen befreit und analysiert.

Die so hergestellten nachvernetzten Polymerpartikel hatten folgende Eigenschaften:

CRC = 30,7 g/g
AUL0.7psi = 24,2 g/g
SFC = $35x10^{-7}cm^3s/g$

| Farbzahl | Messung 1 | Messung 2 | Messung 3 (nach 100 h) | Messung 3 (nach 300 h) |
|---|---|---|---|---|
| L | 93,3 | 89,1 | 80,5 | 81,0 |
| a | -0,8 | -1,5 | 1,0 | 0,1 |
| b | 5,2 | 5,9 | 9,9 | 12,4 |

Beispiel 4

Das in Beispiel 1 hergestellte Grundpolymer (20 g) wurde im Waring-Labormischer auf mittlerer Rührstufe mittels einer Injektionsspritze mit einer Nachvernetzerlösung, bestehend aus 0,02 g N-(2-Hydroxyethyl)-2-oxazolidinon, 0,60 g Wasser und 0,30 g 1,3-Propandiol und 0,20 g Calciumhydroxid dispergiert in dieser Lösung, besprüht. Anschließend wurden 0,48 g einer wässerigen Lösung enthaltend 0,12 g Aluminiumlaktat aufgesprüht. Die feuchten Polymerpartikel wurden mit einem Spatel nochmals homogenisiert und anschließend in einer Petrischale mit einem Innendurchmesser von 18,5 cm gleichmäßig verteilt und in einem Vakuumtrockenschrank (Heraeus VACUTHERM VT 6060M; Kendro Laboratory Products GmbH, DE) für 60 Minuten bei 175 °C wärmebehandelt. Dabei wurde mit ca. 1200 l/h Stickstoff bei ca. 1013 mbar überlagert, wodurch ein Sauerstoffpartialdruck von <10 mbar erzeugt wurde.
Die nachvernetzten Polymerpartikel wurden über ein 850 μm Sieb von Klumpen befreit und analysiert.
Die so hergestellten nachvernetzten Polymerpartikel hatten folgende Eigenschaften:

CRC = 30,4 g/g
AUL0.7psi = 24,5 g/g
SFC = $50x10^{-7}cm^3s/g$

| Farbzahl | Messung 1 | Messung 2 | Messung 3 (nach 100 h) | Messung 3 (nach 300 h) |
|---|---|---|---|---|
| L | 93,2 | 89,3 | 81,0 | 80,9 |
| a | -0,8 | -1,5 | 0,8 | 0,1 |
| b | 5,4 | 6,0 | 9,8 | 12,1 |

Beispiel 5

Das in Beispiel 1 hergestellte Grundpolymer wurde auf 150 bis 700 μm abgesiebt. Das abgesiebte Grundpolymer (20 g) wurde im Waring-Labormischer auf mittlerer Rührstufe mittels einer Injektionsspritze mit einer Nachvernetzerlösung, bestehend aus 0,02 g N-(2-Hydroxyethyl)-2-oxazolidinon, 0,30 g Wasser und 0,30 g 1,3-Propandiol und 0,0006 g Span® 20 besprüht und anschließend wurde 1,3 g wässrige Lösung, enthaltend 0,20 g Calciumlaktat und 0,12 g Aluminiumlaktat, aufgesprüht. Die feuchten Polymerpartikel wurden mit einem Spatel nochmals homogenisiert und anschließend in einer Petrischale mit einem Innendurchmesser von 18,5 cm gleichmäßig verteilt und in einem Vakuumtrockenschrank (Heraeus VACUTHERM VT 6060M; Kendro Laboratory Products GmbH, DE) für 90 Minuten bei 175°C wärmebehandelt. Dabei wurde mit ca. 1200 l/h Stickstoff bei ca. 1013 mbar überlagert. wodurch ein Sauerstoffpartialdruck von <10 mbar erzeugt wurde.
Die nachvernetzten Polymerpartikel wurden über ein 700 μm Sieb von Klumpen befreit und analysiert. Der Anteil an Partikeln kleiner 150 μm betrug weniger als 0,3 Gew.-%.
Die so hergestellten nachvernetzten Polymerpartikel hatten folgende Eigenschaften:

CRC = 29,0 g/g
AUL0.7psi = 24,2 g/g
SFC = $130x10^{-7}cm^3s/g$

| Farbzahl | Messung 1 | Messung 2 | Messung 3 (nach 100 h) | Messung 3 (nach 300 h) |
|---|---|---|---|---|
| L | 90,5 | 86,6 | 80,0 | 78,0 |
| a | -0,9 | -1,2 | 0,1 | 0,2 |
| b | 5,2 | 5,8 | 9,6 | 11,7 |

Beispiel 6

Das in Beispiel 1 hergestellte Grundpolymer wurde auf 150 bis 600 μm abgesiebt. Das abgesiebte Grundpolymer (20 g) wurde im Waring-Labormischer auf mittlerer Rührstufe mittels einer Injektionsspritze mit einer Nachvernetzerlösung, bestehend aus 0,02 g N-(2-Hydroxyethyl)-2-oxazolidinon, 0,30 g Wasser und 0,30 g 1,3-Propandiol und

0,0006 g Span® 20 besprüht und anschließend wurde 1,3 g wässrige Lösung, enthaltend 0,20 g Calciumlaktat und 0,12 g Aluminiumlaktat, aufgesprüht. Die feuchten Polymerpartikel wurden mit einem Spatel nochmals homogenisiert und anschließend in einer Petrischale mit einem Innendurchmesser von 18,5 cm gleichmäßig verteilt und in einem Vakuumtrockenschrank (Heraeus VACUTHERM VT 6060M; Kendro Laboratory Products GmbH, DE) für 90 Minuten bei 175 °C wärmebehandelt. Dabei wurde mit ca. 1200 l/h Stickstoff bei ca. 1013 mbar überlagert. wodurch ein Sauerstoffpartialdruck von <10 mbar erzeugt wurde.

Die nachvernetzten Polymerpartikel wurden über ein 600 $\mu$m Sieb von Klumpen befreit und analysiert. Der Anteil an Partikeln kleiner 150 $\mu$m betrug weniger als 0,3 Gew.-%.

Die so hergestellten nachvernetzten Polymerpartikel hatten folgende Eigenschaften:

CRC = 28,2 g/g
AU L0.7psi = 24,4 g/g
SFC = $115 \times 10^{-7} cm^3 s/g$

| Farbzahl | Messung 1 | Messung 2 | Messung 3 (nach 100 h) | Messung 3 (nach 300 h) |
|---|---|---|---|---|
| L | 91,4 | 87,6 | 81,0 | 79,5 |
| a | -0,7 | -1,0 | 0;2 | 0,2 |
| b | 5,1 | 6,0 | 9,1 | 11,3 |

Beispiel 7 (Vergleichsbeispiel)

Das in Beispiel 1 hergestellte Grundpolymer wurde auf 150 bis 600 $\mu$m abgesiebt. Das abgesiebte Grundpolymer (20 g) wurde im Waring-Labormischer auf mittlerer Rührstufe mittels einer Injektionsspritze mit einer Nachvernetzerlösung, bestehend aus 0,02 g N-(2-Hydroxyethyl)-2-oxazolidinon, 0,30 g Wasser und 0,30 g 1,3-Propandiol und 0,0006 g Span® 20 besprüht und anschließend wurde 1,3 g wässrige Lösung, enthaltend 0,05 g Aluminiumsulfat, aufgesprüht. Die feuchten Polymerpartikel wurden mit einem Spatel nochmals homogenisiert und anschließend in einer Petrischale mit einem Innendurchmesser von 18,5 cm gleichmäßig verteilt und in einem Vakuumtrockenschrank (Heraeus VACUTHERM VT 6060M; Kendro Laboratory Products GmbH, DE) für 90 Minuten bei 175°C wärmebehandelt. Dabei wurde mit ca. 1200 l/h Stickstoff bei ca. 1013 mbar überlagert. wodurch ein Sauerstoffpartialdruck von <10 mbar erzeugt wurde.

Die nachvernetzten Polymerpartikel wurden über ein 600 $\mu$m Sieb von Klumpen befreit und analysiert. Der Anteil an Partikeln kleiner 150 $\mu$m betrug weniger als 0,3 Gew.-%.

Die so hergestellten nachvernetzten Polymerpartikel hatten folgende Eigenschaften:

CRC = 25,9 g/g
AUL0.7psi = 22,2 g/g
SFC = $97 \times 10^{-7} cm^3 s/g$

| Farbzahl | Messung 1 | Messung 2 | Messung 3 (nach 100 h) | Messung 3 (nach 300 h) |
|---|---|---|---|---|
| L | 93,3 | 89,7 | 69,0 | 62,8 |
| a | -1,1 | -1,8 | 2,2 | 3,5 |
| b | 5,5 | 6,1 | 15,1 | 18,7 |

Beispiel 8 (Vergleichsbeispiel)

Das in Beispiel 1 hergestellte Grundpolymer wurde auf 150 bis 600 $\mu$m abgesiebt. Das abgesiebte Grundpolymer (20 g) wurde im Waring-Labormischer auf mittlerer Rührstufe mittels einer Injektionsspritze mit einer Nachvernetzerlösung, bestehend aus 0,02 g N-(2-Hydroxyethyl)-2-oxazolidinon, 0,30 g Wasser und 0,30 g 1,3-Propandiol und 0,0006 g Span® 20 besprüht und anschließend wurden nacheinander 0,7 g wässrige Dispersion, enthaltend 0,05 g Calciumhydroxid, und 0,6 g wässrige Lösung, enthaltend 0,05 g Aluminiumsulfat, aufgesprüht. Die feuchten Polymerpartikel wurden mit einem Spatel nochmals homogenisiert und anschließend in einer Petrischale mit einem Innendurchmesser von 18,5 cm gleichmäßig verteilt und in einem Vakuumtrockenschrank (Heraeus VACUTHERM VT 6060M; Kendro Laboratory Products GmbH, DE) für 90 Minuten bei 175 °C wärmebehandelt. Dabei wurde mit ca. 1200 l/h Stickstoff bei ca. 1013 mbar überlagert. wodurch ein Sauerstoffpartialdruck von <10 mbar erzeugt wurde.

Die nachvernetzten Polymerpartikel wurden über ein 600 μm Sieb von Klumpen befreit und analysiert. Der Anteil an Partikeln kleiner 150 μm betrug weniger als 0,3 Gew.-%.

Die so hergestellten nachvernetzten Polymerpartikel hatten folgende Eigenschaften:

CRC = 26,5 g/g
AUL0.7psi = 22,0 g/g
SFC = $100x10^{-7}cm^3s/g$

| Farbzahl | Messung 1 | Messung 2 | Messung 3 (nach 100 h) | Messung 3 (nach 300 h) |
|---|---|---|---|---|
| L | 91,8 | 87,4 | 80,3 | 79,0 |
| a | -0,8 | -1,2 | 0,4 | 0,3 |
| b | 5,2 | 6,3 | 9,8 | 11,9 |

Beispiel 9 (Vergleichsbeispiel)

Das in Beispiel 1 hergestellte Grundpolymer (20 g) wurde im Waring-Labormischer auf mittlerer Rührstufe mittels einer Injektionsspritze mit einer Nachvernetzerlösung, bestehend aus 0,02 g N-(2-Hydroxyethyl)-2-oxazolidinon, 0,60 g Wasser und 0,30 g 1,3-Propandiol sowie 0,04 g Aluminiumsulfat, besprüht. Die feuchten Polymerpartikel wurden mit einem Spatel nochmals homogenisiert und anschließend in einer Petrischale mit einem Innendurchmesser von 18,5 cm gleichmäßig verteilt und in einem Vakuumtrockenschrank (Heraeus VACUTHERM VT 6060M; Kendro Laboratory Products GmbH, DE) für 60 Minuten bei 175°C wärmebehandelt. Dabei wurde mit ca. 1200 l/h Stickstoff bei ca. 1013 mbar überlagert, wodurch ein Sauerstoffpartialdruck von <10 mbar erzeugt wurde.

Die nachvernetzten Polymerpartikel wurden über ein 850 μm Sieb von Klumpen befreit und analysiert.

Die so hergestellten nachvernetzten Polymerpartikel hatten folgende Eigenschaften:

CRC = 30,8 g/g
AUL0.7psi = 23,2 g/g
SFC = $28x10^{-7}cm^3s/g$

| Farbzahl | Messung 1 | Messung 2 | Messung 3 (nach 100 h) | Messung 3 (nach 300 h) |
|---|---|---|---|---|
| L | 89,8 | 84,9 | 76,9 | 69,0 |
| a | -0,5 | -1,0 | 2,5 | 4,6 |
| b | 8,0 | 8,4 | 11,5 | 16,1 |

Beispiel 10 (Vergleichsbeispiel)

Das in Beispiel 1 hergestellte Grundpolymer (20 g) wurde im Waring-Labormischer auf mittlerer Rührstufe mittels einer Injektionsspritze mit einer Nachvernetzerlösung, bestehend aus 0,02 g N-(2-Hydroxyethyl)-2-oxazolidinon, 0,80 g Wasser und 0,30 g 1,3-Propandiol und 0,10 g Aluminiumlactat, besprüht. Die feuchten Polymerpartikel wurden mit einem Spatel nochmals homogenisiert und anschließend in einer Petrischale mit einem Innendurchmesser von 18,5 cm gleichmäßig verteilt und in einem Vakuumtrockenschrank (Heraeus VACUTHERM VT 6060M; Kendro Laboratory Products GmbH, DE) für 60 Minuten bei 175 °C wärmebehandelt. Dabei wurde mit ca. 1200 l/h Stickstoff bei ca. 1013 mbar überlagert, wodurch ein Sauerstoffpartialdruck von <10 mbar erzeugt wurde.

Die nachvernetzten Polymerpartikel wurden über ein 850 μm Sieb von Klumpen befreit und analysiert.

Die so hergestellten nachvernetzten Polymerpartikel haben folgende Eigenschaften:

CRC = 30,1 g/g
AUL0.7psi = 24,7 g/g

SFC = $47x10^{-7}cm^3s/g$

| Farbzahl | Messung 1 | Messung 2 | Messung 3 (nach 100 h) | Messung 3 (nach 300 h) |
|---|---|---|---|---|
| L | 93,3 | 89,8 | 80,0 | 79,8 |
| a | -1,1 | -1,8 | 0,9 | 0,3 |
| b | 5,5 | 6,1 | 9,2 | 14,7 |

**Patentansprüche**

1. Verfahren zur Herstellung wasserabsorbierender Polymerpartikel, wobei eine Monomerlösung oder -suspension, enthaltend

   a) mindestens eine ungesättigte Carbonsäure, wobei die ungesättigte Carbonsäure zumindest teilweise neutralisiert sein kann, und
   b) mindestens einen Hydrochinonhalbether,

   polymerisiert wird und die Polymerpartikel mit mindestens einem Salz eines dreiwertigen Metallkations beschichtet werden, **dadurch gekennzeichnet, dass** das Salz des dreiwertigen Metallkations das Salz einer Carbonsäure und/oder ein basisches Salz ist und **dadurch gekennzeichnet, dass** die Polymerpartikel mit mindestens einem basischen Salz eines zweiwertigen Metallkations beschichtet werden.

2. Verfahren gemäß Anspruch 1, **dadurch gekennzeichnet, dass** die ungesättigte Carbonsäure a) zu mindestens 25 mol-% neutralisiert ist.

3. Verfahren gemäß Anspruch 1 oder 2, **dadurch gekennzeichnet, dass** die Monomerlösung, bezogen auf die ungesättigte Carbonsäure a), von 0,001 bis 0,016 Gew.-% Hydrochinonhalbether b) enthält.

4. Verfahren gemäß einem der Ansprüche 1 bis 3, **dadurch gekennzeichnet, dass** das Salz des dreiwertigen Metallkations das Salz einer 2-Hydroxycarbonsäure ist.

5. Verfahren gemäß Anspruch 4, **dadurch gekennzeichnet, dass** das zweiwertige Metallkation ein Metallkation der zweiten Hauptgruppe des Periodensystems der Elemente ist.

6. Verfahren gemäß Anspruch 4 oder 5, **dadurch gekennzeichnet, dass** die Polymerpartikel mit einer Lösung oder Suspension des basischen Salzes des zweiwertigen Metallkations beschichtet werden.

7. Verfahren gemäß einem der Ansprüche 4 bis 6, **dadurch gekennzeichnet, dass** die Monomerlösung mindestens ein anorganisches Peroxid enthält.

8. Verfahren gemäß Anspruch 7, **dadurch gekennzeichnet, dass** das anorganische Peroxid c) ein Salz ist.

9. Verfahren gemäß einem der Ansprüche 1 bis 8, **dadurch gekennzeichnet, dass** die Polymerpartikel thermisch nachvernetzt werden.

10. Verfahren gemäß Anspruch 9, **dadurch gekennzeichnet, dass** die Polymerpartikel vor der thermischen Nachvernetzung mit dem Salz des dreiwertigen Metallkations beschichtet werden.

11. Verfahren gemäß Anspruch 9 oder 10, **dadurch gekennzeichnet, dass** die Polymerpartikel vor der thermischen Nachvernetzung mit dem basischen Salz des zweiwertigen Metallkations beschichtet werden.

12. Verfahren gemäß einem der Ansprüche 9 bis 11, **dadurch gekennzeichnet, dass** der Gesamtpartialdruck eines oder mehrerer oxidierender Gase in der die Polymerpartikel während der thermischen Nachvernetzung überlagernden Atmosphäre weniger als 140 mbar beträgt.

13. Verfahren gemäß einem der Ansprüche 9 bis 12, **dadurch gekennzeichnet, dass** grobkörnige Polymerpartikel vor der thermischen Nachvernetzung mittels eines Siebes mit einer Maschenweite von weniger als 700 μm abgetrennt werden.

14. Verfahren gemäß einem der Ansprüche 9 bis 12, **dadurch gekennzeichnet, dass** mindestens ein 1,3-Diol als Nachvernetzer verwendet wird.

15. Verfahren gemäß einem der Ansprüche 9 bis 13, **dadurch gekennzeichnet, dass** mindestens ein cyclisches Carbamat als Nachvernetzer verwendet wird.

16. Wasserabsorbierende Polymerpartikel, herstellbar gemäß einem der Verfahren der Ansprüche 1 bis 15.

17. Polymerpartikel gemäß Anspruch 16, wobei mindestens 90 Gew.-% der Polymerpartikel eine Partikelgröße im Bereich von größer 150 bis 600 μm aufweisen.

18. Polymerpartikel gemäß Anspruch 16 oder 17, wobei die Polymerpartikel nach 300 Stunden bei 60 °C und einer relativen Luftfeuchtigkeit von 90 % einen b-Wert von nicht mehr als 15 aufweisen.

19. Hygieneartikel, enthaltend wasserabsorbierende Polymerpartikel gemäß einem der Ansprüche 16 bis 18.

## Claims

1. A process for producing water-absorbing polymeric particles by polymerizing a monomer solution or suspension comprising

a) at least one unsaturated carboxylic acid, which may be at least partially neutralized, and
b) at least one hydroquinone monoether

and coating the polymeric particles with at least one salt of a tervalent metal cation wherein the salt of the tervalent metal cation is the salt of a carboxylic acid and/or a basic salt and wherein the polymeric particles are coated with at least one basic salt of a bivalent metal cation.

2. The process according to claim 1 wherein the unsaturated carboxylic acid a) is at least 25 mol% neutralized.

3. The process according to claim 1 or 2 wherein the monomer solution comprises from 0.001% to 0.016% by weight of hydroquinone monoether b), based on the unsaturated carboxylic acid a).

4. The process according to any one of the claims 1 to 3 wherein the salt of the tervalent metal cation is the salt of a 2-hydroxy carboxylic acids.

5. The process according to claim 4 wherein the bivalent metal cation is a metal cation of the second main group of the periodic table.

6. The process according to claim 4 or 5 wherein the polymeric particles are coated with a solution or suspension of the basic salt of the bivalent metal cation.

7. The process according to any one of the claims 4 to 6 wherein the monomer solution comprises at least one inorganic peroxide.

8. The process according to claim 7 wherein the inorganic peroxide c) is a salt.

9. The process according to any one of the claims 1 to 8 wherein the polymeric particles are thermally postcrosslinked.

10. The process according to claim 9 wherein the polymeric particles are coated with the salt of the tervalent metal cation prior to thermal postcrosslinking.

11. The process according to claim 9 or 10 wherein the polymeric particles are coated with the basic salt of the bivalent

metal cation prior to thermal postcrosslinking.

**12.** The process according to any one of the claims 9 to 11 wherein the total partial pressure of one or more oxidizing gases in the atmosphere overlying the polymeric particles during thermal postcrosslinking is less than 140 mbar.

**13.** The process according to any one of the claims 9 to 12 wherein coarse polymeric particles are removed before thermal postcrosslinking by means of a sieve having a mesh size of less than 700 μm.

**14.** The process according to any one of the claims 9 to 12 wherein at least one 1,3-diol is used as postcrosslinker.

**15.** The process according to any one of the claims 9 to 13 wherein at least one cyclic carbamate is used as postcrosslinker.

**16.** Water-absorbing polymeric particles obtainable according to any one of the processes of claims 1 to 15.

**17.** The polymeric particles according to claim 16 wherein at least 90% by weight of the polymeric particles have a particle size in the range from greater than 150 to 600 μm.

**18.** The polymeric particles according to claim 16 or 17 wherein the polymeric particles after 300 hours at 60°C and a relative humidity of 90% have a b value of not more than 15.

**19.** A hygiene article comprising water-absorbing polymeric particles according to any one of the claims 16 to 18.

## Revendications

**1.** Procédé de fabrication de particules polymères absorbant l'eau, selon lequel une solution ou suspension de monomères, contenant :

a) au moins un acide carboxylique insaturé, l'acide carboxylique insaturé pouvant au moins partiellement être neutralisé, et
b) au moins un semi-éther d'hydroquinone,

est polymérisée, et les particules polymères sont revêtues avec au moins un sel d'un cation métallique trivalent, **caractérisé en ce que** le sel du cation métallique trivalent est le sel d'un acide carboxylique et/ou un sel basique, et **caractérisé en ce que** les particules polymères sont revêtues avec au moins un sel basique d'un cation métallique bivalent.

**2.** Procédé selon la revendication 1, **caractérisé en ce que** l'acide carboxylique insaturé a) est neutralisé à hauteur d'au moins 25 % en moles.

**3.** Procédé selon la revendication 1 ou 2, **caractérisé en ce que** la solution de monomères contient, par rapport à l'acide carboxylique insaturé a), de 0,001 à 0,016 % en poids de semi-éther d'hydroquinone b).

**4.** Procédé selon l'une quelconque des revendications 1 à 3, **caractérisé en ce que** le sel du cation métallique trivalent est le sel d'un acide 2-hydroxycarboxylique.

**5.** Procédé selon la revendication 4, **caractérisé en ce que** le cation métallique bivalent est un cation métallique du deuxième groupe principal du tableau périodique des éléments.

**6.** Procédé selon la revendication 4 ou 5, **caractérisé en ce que** les particules polymères sont revêtues avec une solution ou suspension du sel basique du cation métallique bivalent.

**7.** Procédé selon l'une quelconque des revendications 4 à 6, **caractérisé en ce que** la solution de monomères contient au moins un peroxyde inorganique.

**8.** Procédé selon la revendication 7, **caractérisé en ce que** le peroxyde inorganique c) est un sel.

**9.** Procédé selon l'une quelconque des revendications 1 à 8, **caractérisé en ce que** les particules polymères sont post-réticulées thermiquement.

**10.** Procédé selon la revendication 9, **caractérisé en ce que** les particules polymères sont revêtues avec le sel du cation métallique trivalent avant la post-réticulation thermique.

**11.** Procédé selon la revendication 9 ou 10, **caractérisé en ce que** les particules polymères sont revêtues avec le sel basique du cation métallique bivalent avant la post-réticulation thermique.

**12.** Procédé selon l'une quelconque des revendications 9 à 11, **caractérisé en ce que** la pression partielle totale d'un ou de plusieurs gaz oxydants dans l'atmosphère recouvrant les particules polymères pendant la post-réticulation thermique est inférieure à 140 mbar.

**13.** Procédé selon l'une quelconque des revendications 9 à 12, **caractérisé en ce que** des particules polymères de taille grossière sont séparées avant la post-réticulation thermique au moyen d'un tamis d'une largeur de maille inférieure à 700 μm.

**14.** Procédé selon l'une quelconque des revendications 9 à 12, **caractérisé en ce qu'**au moins un 1,3-diol est utilisé en tant qu'agent de post-réticulation.

**15.** Procédé selon l'une quelconque des revendications 9 à 13, **caractérisé en ce qu'**au moins un carbamate cyclique est utilisé en tant qu'agent de post-réticulation.

**16.** Particules polymères absorbant l'eau, pouvant être fabriquées par un des procédés selon les revendications 1 à 15.

**17.** Particules polymères selon la revendication 16, dans lesquelles au moins 90 % en poids des particules polymères présentent une taille de particule dans la plage allant de plus de 150 à 600 μm.

**18.** Particules polymères selon la revendication 16 ou 17, dans lesquelles les particules polymères présentent, après 300 heures à 60 °C et une humidité relative de l'air de 90 %, une valeur b non supérieure à 15.

**19.** Article d'hygiène, contenant des particules polymères absorbant l'eau selon l'une quelconque des revendications 16 à 18.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006015729 A2
- WO 2000053664 A1
- DE 102004057874 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- EP 0343427 A2
- DE 19941423 A1
- EP 0686650 A1
- WO 200145758 A1
- WO 200138402 A1
- EP 0955086 A2
- EP 0083022 A2
- EP 0543303 A1
- EP 0937736 A2
- DE 3314019 A1
- DE 3523617 A1
- EP 0450922 A2
- DE 10204938 A1
- US 6239230 B
- EP 1199327 A2
- DE 4020780 C1
- DE 19807502 A1
- DE 19807992 C1
- DE 19854573 A1
- DE 19854574 A1
- DE 10204937 A1
- DE 10334584 A1
- WO 2003031482 A1
- EP 0534228 A1
- EP 1191051 A2
- US 20030181115 A
- US 20040019342 A
- EP 0377199 A2
- EP 0445641 A1
- US 5026806 A
- EP 0655465 A1
- EP 0377191 A2
- EP 0640330 A1


### In der Beschreibung aufgeführte Nicht-Patentliteratur


- **F.L. BUCHHOLZ ; A.T. GRAHAM.** Modern Superabsorbent Polymer Technology. Wiley-VCH, 1998, 97-103
- **F.L. BUCHHOLZ ; A.T. GRAHAM.** Modern Superabsorbent Polymer Technology. Wiley-VCH, 1998
- Ullmann's Encyclopedia of Industrial Chemistry. vol. 35, 73-103
- Zerstäuben von Flüssigkeiten. **THOMAS RICHTER.** Reihe Kontakt & Studium. Expert-Verlag, 2004, vol. 660
- Zerstäubungstechnik. **GÜNTER WOZNIAK.** VDI-Reihe. Springer-Verlag, 2002
- *Hunterlab,* 1996, vol. 8, 1-4